(19) Europäisches Patentamt
European Patent Office
Office européen des brevets

(11) EP 4 591 887 A1

(12) EUROPEAN PATENT APPLICATION
published in accordance with Art. 153(4) EPC

(43) Date of publication:
**30.07.2025 Bulletin 2025/31**

(21) Application number: **23867590.4**

(22) Date of filing: **21.09.2023**

(51) International Patent Classification (IPC):
***A61K 47/68*** *(2017.01)* ***A61K 45/00*** *(2006.01)*
***A61P 35/00*** *(2006.01)*

(52) Cooperative Patent Classification (CPC):
**A61K 45/00; A61K 47/68; A61P 35/00**

(86) International application number:
**PCT/CN2023/120286**

(87) International publication number:
**WO 2024/061305 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.09.2022 CN 202211156361**

(71) Applicant: **Lepu Biopharma Co., Ltd.**
**Shanghai 201114 (CN)**

(72) Inventors:
- **WANG, Yanchun**
  **Shanghai 201203 (CN)**
- **SHEN, Hao**
  **Shanghai 201108 (CN)**
- **LIU, Wenchao**
  **Shanghai 201203 (CN)**
- **CUI, Feifei**
  **Shanghai 201108 (CN)**
- **LIU, Shoujia**
  **Shanghai 201203 (CN)**
- **YANG, Liu**
  **Shanghai 201108 (CN)**
- **LI, Hongfeng**
  **Shanghai 201203 (CN)**
- **DAI, Zhenyu**
  **Shanghai 201203 (CN)**
- **FANG, Lei**
  **Shanghai 201108 (CN)**
- **LI, Hu**
  **Shanghai 201203 (CN)**
- **HU, Chaohong**
  **Shanghai 201203 (CN)**

(74) Representative: **Ipsilon**
**11 rue Saint-Georges**
**75009 Paris (FR)**

Remarks:
The complete document including Reference Table(s) and the Sequence Listing(s) can be downloaded from the EPO website

# (54) GPC3 ANTIBODY-DRUG CONJUGATE AND USE THEREOF

(57) The present invention provides an antibody-drug conjugate and use thereof. The antibody-drug conjugate has a structure represented by formula I, namely Ab-(L-D)p, wherein Ab is an anti-GPC3 antibody, L is a linker, and D is a cytotoxin. The antibody-drug conjugate of the present invention has good activity in inhibiting tumor cell growth *in vivo* and *in vitro,* and has relatively low cytotoxicity.

EP 4 591 887 A1

## Description

### Technical Field

The present invention relates to the field of pharmaceutical chemistry, and in particular to a GPC3 antibody-drug conjugate and the use thereof.

### Background Art

Liver cancer is the fifth most prevalent cancer in the world and also the third most common cause of cancer-related death. Hepatocellular carcinoma (HCC) is the main form of liver cancer and accounts for 90% of all liver cancers. At present, a well-accepted therapeutic scheme is multidisciplinary comprehensive treatment with surgical resection as the core. Although there is an improved short-term curative effect, the long-term curative effect is still not ideal. Therefore, exploring a more effective new strategy for treating liver cancer is currently a research hotspot at home and abroad, and it is still urgent to develop a new drug for treating liver cancer.

GPC3, with its full name of Glypican-3, is a member of the heparan sulfate proteoglycan family, and is anchored to the cell surface via glycosyl phosphatidylinositol on the cell membrane. The protein core of GPC3 consists of two subunits, wherein the N-terminal subunit is about 40 kDa in size, and the C-terminal subunit is about 30 kDa in size. Six Glypicans (GPC1-6) have been identified in mammals. GPC3 plays an important role in regulating cell proliferation, differentiation, adhesion, and migration. It is believed in some studies that GPC3 interacts with Wnt protein and Frizzled receptor to form a complex and trigger downstream signaling. The core protein of GPC3 can act as a Wnt co-receptor or receiver.

In the human body, the expression of GPC3 protein expressed from the *GPC3* gene varies significantly in different development stages and different tissues. For example, the content of the protein in normal adult tissues is very small, and the protein is under-expressed or not expressed in cancers such as gastric cancer, breast cancer, and ovarian cancer but often over-expressed (70-80%) in hepatocellular carcinoma (HCC). Studies have shown that GPC3 is a liver cancer-specific associated antigen, which can help to confirm the nature of lesions in the early stage of HCC and can significantly improve the accuracy of diagnosis. Therefore, GPC3 is considered to have great potential in immunotherapy for liver cancer.

In recent years, GPC3 targeted therapy has attracted increasing attention. At present, the therapeutic strategies based on GPC3 target mainly focus on antibody drugs, cell therapies, vaccines, etc. Therapeutic anti-GPC3 antibodies have been developed, including antibodies such as GC33, YP7 and HN3 and bispecific antibodies (e.g., ERY974 and CM350 under clinical trials). Some of these antibodies inhibit Wnt signaling in liver cancer cells. In addition, chimeric antigen receptor (CAR) T cell immunotherapy has been developed at various stages for treating liver cancer. In mice with xenograft or orthotopic liver tumors, CAR-T cells can eliminate GPC3-positive cancer cells by inducing cell death mediated by perforin and granzyme and reducing WNT signaling in tumor cells.

### Summary of the Invention

Through a lot of experiments and creative effort, the inventors of the present application prepared an anti-GPC3 antibody-drug conjugate (ADC) and confirmed that it has good biological activity, thus completing the present invention.

To this end, in a first aspect of the present invention, the present invention provides an antibody-drug conjugate, or a pharmaceutically acceptable salt or solvate thereof, or a solvate of the salt, wherein the antibody-drug conjugate has a structure represented by formula I,

$$Ab\text{-}(L\text{-}D)_p \qquad \text{formula I}$$

in which:

Ab is an anti-GPC3 antibody, and the anti-GPC3 antibody comprises a heavy chain and a light chain, wherein CDR1, CDR2 and CDR3 in a heavy chain variable region comprise the sequences as set forth in SEQ ID NO: 1, SEQ ID NO: 2 and SEQ ID NO: 3, respectively, and CDR1, CDR2 and CDR3 in a light chain variable region comprise the sequences as set forth in SEQ ID NO: 9, SEQ ID NO: 10 and SEQ ID NO: 11, respectively;
L is a linker;
D is a cytotoxin; and
p is any numerical value between 1 and 8 (e.g., 1, 1.5, 2, 2.5, 3, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, or 8, or e.g., 1-1.5, 1.5-2, 2-2.5, 2.5-3, 3-3.5, 3.5-4, 4-4.5, 4.5-5, 5-5.5, 5.5-6, 6-6.5, 6.5-7, 7-7.5, or 7.5-8).

In formula I, L-D means that the linker is linked to the cytotoxin via a covalent bond to form an L-D molecule; and Ab-$(L-D)_p$ means that p L-D molecules are conjugated to Ab via covalent bonds.

In the present invention, the drug-to-antibody ratio (DAR) refers to the number of drug molecules conjugated to the antibody (e.g., p in formula I). The number of drug molecules contained in the antibody-drug conjugate described herein can be either an integer or a decimal. The number, whether an integer or a decimal, refers to the average number of drug molecules conjugated to each antibody. "p is any numerical value between 1 and 8" means that p may be either any integer between 1 and 8 (including endpoints 1 and 8) or any decimal between 1 and 8, e.g., 2.3, 3.9, 4.0, or 4.2. Moreover, those skilled in the art can understand that even if the same preparation method is used, the DAR values of antibody-drug conjugates prepared in different batches are not necessarily the same, for example, they can fluctuate within a range of no more than 0.5 up and down.

The drug-to-antibody ratio (DAR) can be verified by conventional means, such as mass spectrometry, ELISA assay, HIC, and HPLC. ADCs may also be measured for quantitative distribution in terms of p. In some cases, the separation of homogeneous ADCs with p as a certain numerical value from ADCs with other drug loads, followed by purification and verification can be achieved by means such as reversed-phase HPLC or electrophoresis.

In some embodiments, the linker is selected from: 6-maleimidocaproyl (MC), maleimidopropionyl (MP), N-succinimidyl 4-(2-pyridylthio)pentanoate (SPP), 4-(N-maleimidomethyl)-cyclohexane-1-formyl (MCC), N-succinimidyl (4-iodoacetyl)aminobenzoate (SIAB), 6-maleimidocaproyl-valine-citrulline-p-aminobenzyloxycarbonyl (MC-vc-PAB), MA-PEG4-VC-PAB-DMEA, and

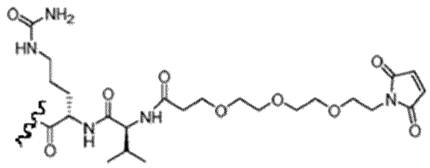

.

In some embodiments, the linker is selected from: 6-maleimidocaproyl (MC), 6-maleimidocaproyl-valine-citrulline-p-aminobenzyloxycarbonyl (MC-vc-PAB), MA-PEG4-VC-PAB-DMEA, and

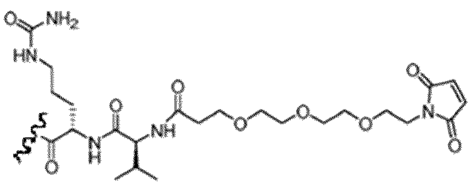

.

The structural formulas of MC, MC-vc-PAB, and MA-PEG4-VC-PAB-DMEA are as shown below:

| | |
|---|---|
| MC | 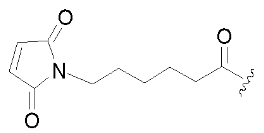 |
| MC-vc-PAB | 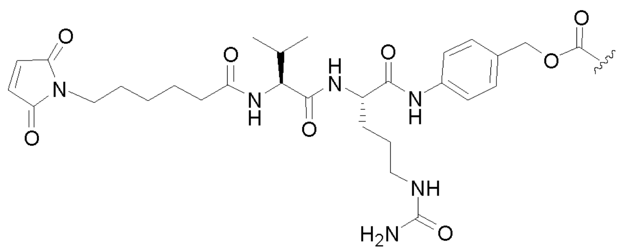 |

(continued)

| | |
|---|---|
| MA-PEG4-VC-PAB-DMEA | 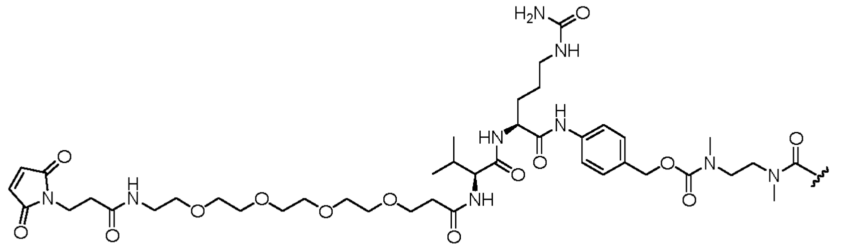 |

In some embodiments, the cytotoxin is selected from: SN-38, gemcitabine, monomethyl auristatin E (MMAE), monomethyl auristatin F (MMAF), maytansinoids (e.g., maytansine DM1 and maytansine DM4), calicheamicin, MGBAs (e.g., duocarmycin), doxorubicin, ricin, diphtherin, duocarmycin SA,

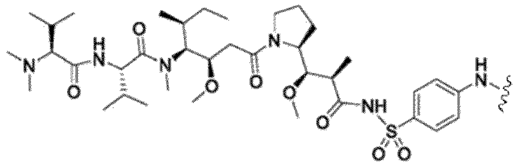

,

1131, interleukins, tumor necrosis factors, chemokines, and nanoparticles.

In some embodiments, the cytotoxin is selected from: monomethyl auristatin E (MMAE), duocarmycin SA, monomethyl auristatin F (MMAF), and

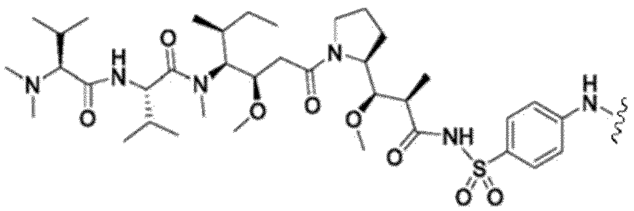

.

The structural formulas of MMAE, MMAF, and duocarmycin SA are as shown below:

| | |
|---|---|
| MMAE | 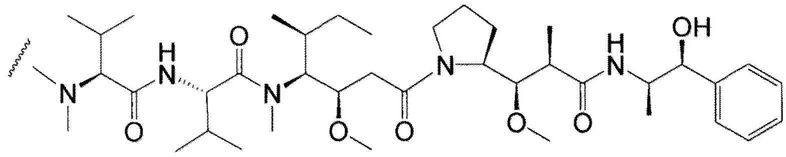 |
| MMAF | 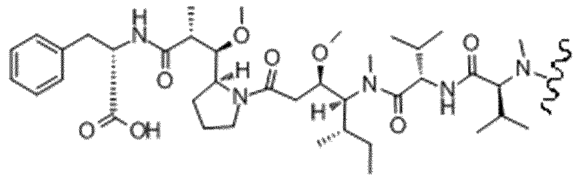 |
| Duocarmycin SA | 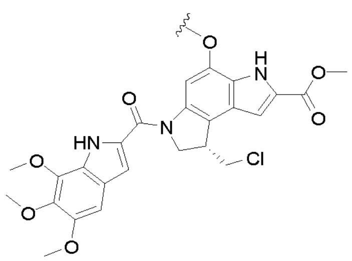 |

In some embodiments, L-D is selected from: MC-vc-PAB-MMAE, MA-PEG4-VC-PAB-DMEA-Duocarmycin SA, MC-MMAF, and

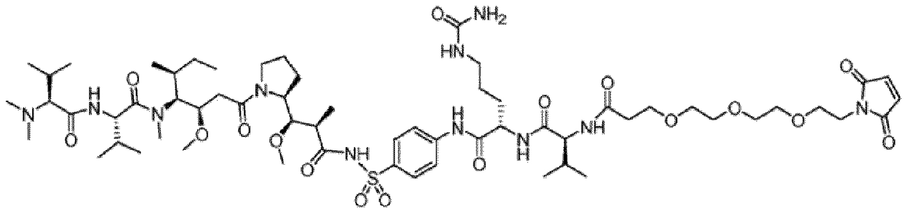

The structural formulas of MC-vc-PAB-MMAE, MA-PEG4-VC-PAB-DMEA-Duocarmycin SA, and MC-MMAF are as shown below:

| | |
|---|---|
| MC-vc-PAB-MMAE | 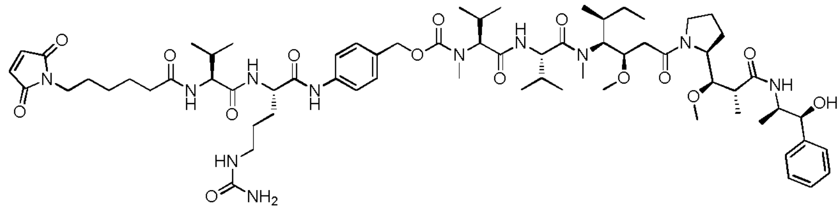 |
| MC-MMAF | 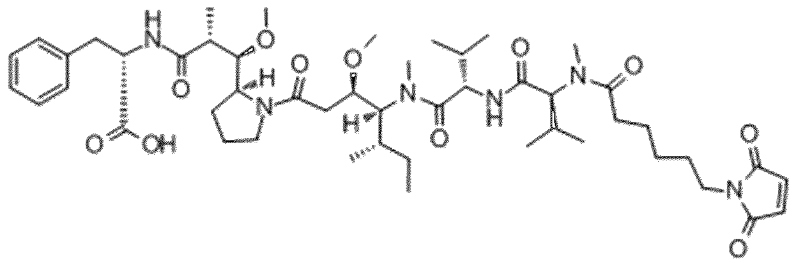 |
| MA-PEG4-VC-PAB-DMEA-Duocarmycin SA | 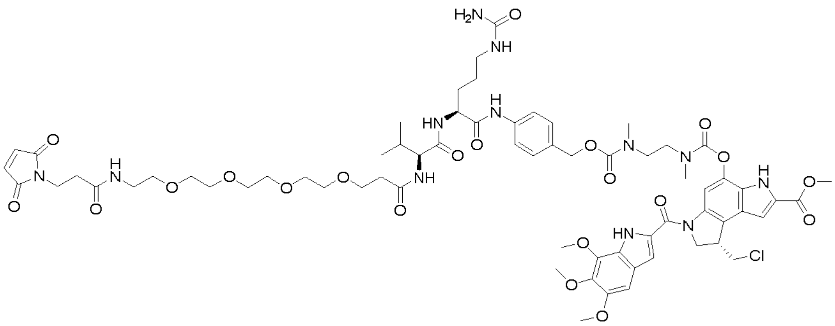 |

When the above four L-Ds are conjugated to Ab via covalent bonds, both the conjugation mode and conjugation site are the same, that is, they are all formed by conjugating succinimide at one end of L-D to mercapto in the antibody. For example, when MC-vc-PAB-MMAE is conjugated to Ab via a covalent bond, the structural formula of the formed ADC is as shown below:

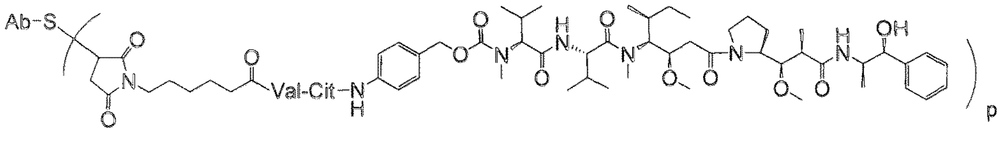

In the formed ADC above, the antibody Ab is linked to a carbon atom of the succinimide at the end of L-D via -S-, and the -S- is not an additionally added mercapto group, but a mercapto group contained in the antibody itself after the antibody Ab is reduced to break a disulfide bond.

In some embodiments, in the anti-GPC3 antibody, the sequences of CDR1, CDR2, and CDR3 in a heavy chain

variable region are as set forth in SEQ ID NO: 1, SEQ ID NO: 2, and SEQ ID NO: 3, respectively, and the sequences of CDR1, CDR2, and CDR3 in a light chain variable region are as set forth in SEQ ID NO: 9, SEQ ID NO: 10, and SEQ ID NO: 11, respectively.

In some embodiments, the sequences of FR1, FR2, FR3, and FR4 regions in a heavy chain variable region of the anti-GPC3 antibody are as set forth in SEQ ID NO: 4, SEQ ID NO: 5, SEQ ID NO: 6, and SEQ ID NO: 7, respectively.

In some embodiments, the sequences of FR1, FR2, FR3, and FR4 regions in a light chain variable region of the anti-GPC3 antibody are as set forth in SEQ ID NO: 12, SEQ ID NO: 13, SEQ ID NO: 14, and SEQ ID NO: 15, respectively, or the sequences of FR1, FR2, FR3, and FR4 regions in a light chain variable region of the anti-GPC3 antibody are as set forth in SEQ ID NO: 12, SEQ ID NO: 13, SEQ ID NO: 17, and SEQ ID NO: 15, respectively.

In some embodiments, the sequence of the heavy chain variable region of the anti-GPC3 antibody is as set forth in SEQ ID NO: 8.

In some embodiments, the sequence of the light chain variable region of the anti-GPC3 antibody is as set forth in SEQ ID NO: 16 or SEQ ID NO: 18.

In some embodiments, the sequence of the heavy chain variable region of the anti-GPC3 antibody is as set forth in SEQ ID NO: 8, and the sequence of the light chain variable region of the anti-GPC3 antibody is as set forth in SEQ ID NO: 16.

In some embodiments, the sequence of the heavy chain variable region of the anti-GPC3 antibody is as set forth in SEQ ID NO: 8, and the sequence of the light chain variable region of the anti-GPC3 antibody is as set forth in SEQ ID NO: 18.

In some embodiments, a heavy chain constant region of the anti-GPC3 antibody is selected from human IgG, IgM, IgA, IgD, and IgE constant regions, or mutants of the constant regions.

In some embodiments, the IgG is selected from IgG1, IgG2, IgG3, and IgG4.

In some embodiments, a light chain constant region of the anti-GPC3 antibody is human lambda constant region or kappa constant region, or a mutant of the constant region.

In some embodiments, the sequence of the heavy chain constant region of the anti-GPC3 antibody comprises the sequence as set forth in SEQ ID NO: 19, or a sequence with more than 70%, preferably more than 75%, 80%, 85%, 90%, 95%, or 99% homology with the sequence as set forth in SEQ ID NO: 19.

In some embodiments, the sequence of the heavy chain constant region of the anti-GPC3 antibody is as set forth in SEQ ID NO: 19.

In some embodiments, the sequence of the light chain constant region of the anti-GPC3 antibody comprises the sequence as set forth in SEQ ID NO: 20, or a sequence with more than 70%, preferably more than 75%, 80%, 85%, 90%, 95%, or 99% homology with the sequence as set forth in SEQ ID NO: 20.

In some embodiments, the sequence of the light chain constant region of the anti-GPC3 antibody is as set forth in SEQ ID NO: 20.

In some embodiments, p is any numerical value between 2 and 8.

In some embodiments, p is any numerical value between 2 and 6.

In some embodiments, p is any numerical value between 2 and 5 (e.g., 2.3, 3.9, 4.0, or 4.2).

In a second aspect of the present invention, the present invention provides a pharmaceutical composition comprising the aforementioned antibody-drug conjugate, or the pharmaceutically acceptable salt or solvate thereof, or the solvate of the salt.

In some embodiments, the pharmaceutical composition further comprises at least one of a chemotherapeutic drug, an immunotherapeutic drug, and an immunosuppressant for treating a tumor.

In some embodiments, the chemotherapeutic drug is, for example, doxorubicin (Adriamycin), cyclophosphamide, a taxane (e.g., paclitaxel (Taxol) or docetaxel (Taxotere)), capecitabine (Xeloda), gemcitabine (Gemzar), vinorelbine (Navelbine), tamoxifen, an aromatase inhibitor (Arimidex, Femara, or Aromasin), 5-FU + folinic acid, irinotecan (Camptosar), oxaliplatin, cisplatin, carboplatin, estramustine, mitoxantrone (Novantrone), prednisone, vincristine (Oncovin), adriamycin, prednisone, etc., or a combination thereof.

In some embodiments, the immunotherapeutic drug is, for example, an anti-PD-1 monoclonal antibody (e.g., Pembrolizumab or Nivolumab), an anti-PD-L1 monoclonal antibody (e.g., Atezolizumab), an anti-TIGIT monoclonal antibody, an anti-4-1BB monoclonal antibody, an anti-VEGFR2 monoclonal antibody (e.g., Ramucirumab or apatinib), an anti-HER2 monoclonal antibody (e.g., Trastuzumab, Trastuzumab biosimilar, or Trastuzumab-dkst), etc., or a combination thereof.

In some embodiments, the immunosuppressant is selected from: (1) glucocorticoids, such as cortisone and prednisone; (2) microbial metabolites, such as cyclosporine and Fujimycin; (3) antimetabolites, such as azathioprine and 6-mercaptopurine; (4) polyclonal and monoclonal anti-lymphocyte antibodies, such as anti-lymphocyte globulin and OKT3; and (5) alkylating agents, such as cyclophosphamide. In some specific embodiments, the immunosuppressant is, for example, methylprednisolone, prednisone, azathioprine, Prograf, Zenapax, Simulect, cyclosporine, tacrolimus, rapamycin, mycophenolate mofetil, mizoribine, cyclophosphamide, or fingolimod.

In some embodiments, the pharmaceutical composition further comprises at least one pharmaceutical auxiliary material.

In a third aspect of the present invention, the present invention provides the use of the aforementioned antibody-drug conjugate, or the pharmaceutically acceptable salt or solvate thereof, or the solvate of the salt or the aforementioned pharmaceutical composition in the preparation of a drug for preventing and/or treating a GPC3-associated disease, wherein preferably, the GPC3-associated disease is a GPC3 positivity-associated disease.

In some embodiments, the GPC3 positivity-associated disease includes, is but not limited to, liver cancer, lung cancer, gastric cancer, head and neck cancer, esophageal cancer, Merkel cell cancer, or liposarcoma. In some embodiments, the GPC3 positivity-associated disease is liver cancer.

In a fourth aspect of the present invention, the present invention provides the aforementioned antibody-drug conjugate, or the pharmaceutically acceptable salt or solvate thereof, or the solvate of the salt or the aforementioned pharmaceutical composition, for use in preventing and/or treating a GPC3-associated disease, wherein preferably, the GPC3-associated disease is a GPC3 positivity-associated disease.

In some embodiments, the GPC3 positivity-associated disease includes, is but not limited to, liver cancer, lung cancer, gastric cancer, head and neck cancer, esophageal cancer, Merkel cell cancer, or liposarcoma. In some embodiments, the GPC3 positivity-associated disease is liver cancer.

In a fifth aspect of the present invention, the present invention provides a method for treating and/or preventing a GPC3-associated disease, comprising: administering to a subject in need thereof a therapeutically and/or prophylactically effective amount of the aforementioned antibody-drug conjugate, or the pharmaceutically acceptable salt or solvate thereof, or the solvate of the salt or the aforementioned pharmaceutical composition, wherein preferably, the GPC3-associated disease is a GPC3 positivity-associated disease.

In some embodiments, the GPC3 positivity-associated disease includes, is but not limited to, liver cancer, lung cancer, gastric cancer, head and neck cancer, esophageal cancer, Merkel cell cancer, or liposarcoma. In some embodiments, the GPC3 positivity-associated disease is liver cancer.

## Beneficial Effects

1. The novel antibodies Hu 52H5D3B8-7 and Hu 52H5D3B8-8 of the present invention are developed anti-GPC3 monoclonal antibodies, which can be conjugated to various potent cytotoxic small molecule drugs such as MMAE (monomethyl auristatin E), MMAF, and Duo to form GPC3-ADCs via a linker. The GPC3-ADCs can effectively inhibit tumor cell proliferation and lead to tumor cell death by binding to GPC3 receptor on the tumor cell surface, endocytosis, and release of small molecular toxins.
2. Compared with known antibodies such as Y035 and GC33, the novel antibodies Hu 52H5D3B8-7 and Hu 52H5D3B8-8 of the present invention have stronger cell binding affinity and better internalization effect. In addition, after conjugation with cytotoxin, the affinity of the antibodies for HepG2 cells expressing GPC3 is not changed.
3. The newly developed antibodies Hu 52H5D3B8-7 and Hu 52H5D3B8-8 of the present invention can be conjugated to a variety of different linker-payloads and the conjugates exhibit a relatively strong cell killing effect in liver cancer cells, which is significantly stronger than that of GC33-ADCs with the same linker-payloads.
4. The newly developed GPC3-ADCs of the present invention exhibit a significant pharmacodynamic effect on inhibiting tumor cell growth in a PDX tumor model of human liver cancer.

## Brief Description of the Drawings

Figure 1 shows the affinity of each GPC3 antibody for GPC3-CHO-K1 cells;
Figure 2 shows the affinity of each GPC3 antibody for HepG2 cells;
Figure 3 shows the affinity of each GPC3 antibody for Huh-7 cells;
Figure 4 shows the internalization of each GPC3 antibody on HepG2 cells;
Figure 5 shows a hydrophobic interaction chromatogram of Hu 52H5D3B8-7-L1-D1;
Figure 6 shows a hydrophobic interaction chromatogram of Hu 52H5D3B8-7-L3-D3;
Figure 7 shows a hydrophobic interaction chromatogram of Hu 52H5D3B8-7-L4-D4;
Figure 8 shows a hydrophobic interaction chromatogram of Hu 52H5D3B8-8-ZLA;
Figure 9 shows the affinity of each GPC3 antibody and ADC for HepG2 cells;
Figure 10 shows the cell killing effect of Hu 52H5D3B8-8-L1-D1 and GC33-L1-D1 in an Huh7 cell line;
Figure 11 shows the cell killing effect of Hu 52H5D3B8-8-L1-D1 and GC33-L1-D1 in an HepG2 cell line;

Figure 12 shows the cell killing effect of Hu 52H5D3B8-8-L3-D3 and GC33-L3-D3 in an Huh7 cell line;
Figure 13 shows the cell killing effect of Hu 52H5D3B8-7-L4-D4 and GC33-L4-D4 in an Huh7 cell line;
Figure 14 shows the cell killing effect of Hu 52H5D3B8-8-ZLA and GC33-ZLA in an Huh7 cell line;
Figure 15 shows the cell killing effect of Hu 52H5D3B8-8-ZLA and GC33-ZLA in an HepG2 cell line;
Figure 16 shows the inhibitory activity of each GPC3-ADC on tumor growth in PDX model LIV#219 of nude mice with human liver cancer; and
Figure 17 shows the effect of each GPC3-ADC on the animal body weight of PDX model LIV#219 of nude mice with human liver cancer.

Detailed Description of Embodiments

Embodiments of the present invention will be described in detail below with reference to examples; however, those skilled in the art will understand that the following examples are only used to illustrate the present invention and should not be regarded as limiting the scope of the present invention. For examples in which specific conditions are not specified, they are carried out according to the conventional conditions or the conditions recommended by the manufacturer. The reagents or instruments used without indicating the manufacturer are all commercially available conventional products.

In the present invention, the scientific and technical terms used herein have the meanings that are commonly understood by those skilled in the art unless otherwise specified. In addition, the terms and laboratory operation steps related to the protein and nucleic acid chemistry, molecular biology, cell and tissue culture, microbiology and immunology used herein are all terms and conventional steps that are widely used in the corresponding art. Moreover, for better understanding of the present invention, definitions and explanations of related terms are provided below.

In the present invention, any numerical range should be interpreted as including any value within the range or any subrange unless otherwise specified.

In the present invention, the term "antibody" refers to an immunoglobulin molecule that generally consists of two pairs of identical polypeptide chains (each pair has one "light" (L) chain and one "heavy" (H) chain). Antibody light chains can be divided into two categories, i.e., $\kappa$ and $\lambda$. Heavy chains can be divided into five types, i.e., $\mu$, $\delta$, $\gamma$, $\alpha$, or $\varepsilon$. According to different heavy chains, antibodies can be divided into five categories, i.e., IgM, IgD, IgG, IgA, and IgE. Within light and heavy chains, the variable and constant regions are joined by a "J" region of about 12 or more amino acids, with the heavy chain further comprising a "D" region of about 3 or more amino acids. Each heavy chain consists of a heavy chain variable region ($V_H$) and a heavy chain constant region ($C_H$). The heavy chain constant region consists of three domains ($C_H1$, $C_H2$, and $C_H3$). Each light chain consists of a light chain variable region ($V_L$) and a light chain constant region ($C_L$). The light chain constant region consists of one domain, i.e., $C_L$. The constant regions of the antibodies can mediate the binding of the immunoglobulin to host tissues or factors, including various cells of the immune system (e.g., effector cells) and component C1q of the complement system. The $V_H$ and $V_L$ regions can be further subdivided into regions of hypervariability, termed complementarity determining regions (CDRs), interspersed with regions that are more conserved, termed framework regions (FRs). Each $V_H$ and $V_L$ consists of three CDRs and four FRs, arranged from amino-terminus to carboxy-terminus in the following order: FR1, CDR1, FR2, CDR2, FR3, CDR3, FR4. The variable regions ($V_H$ and $V_L$) of each heavy/light chain pair form antibody binding sites, respectively. Distribution of amino acids in various regions or domains follows the definitions in: Kabat Sequences of Proteins of Immunological Interest (National Institutes of Health, Bethesda, Md. (1987 and 1991)), or Chothia & Lesk (1987) J. Mol. Biol. 196:901-917; and Chothia et al. (1989) Nature 342:878-883.

In the present invention, algorithms for determining the percentage of sequence homology and sequence similarity are, for example, BLAST and BLAST 2.0 algorithms, which are described in Altschul et al. (1977) Nucl. Acid. Res. 25:3389-3402 and Altschul et al. (1990) J. Mol. Biol. 215:403-410, respectively. BLAST and BLAST 2.0 can be used to determine the percentage of amino acid sequence homology of the present invention, using parameters such as those described in the literatures or default parameters. Softwares for performing BLAST analyses are publicly available through the National Center for Biotechnology Information (NCBI).

In the present invention, the amino acid sequence having at least 70% sequence homology with an amino acid sequence includes a polypeptide sequence essentially identical to the amino acid sequence, e.g., those sequences comprising at least 70% sequence homology, preferably at least 75%, 80%, 85%, 86%, 87%, 88%, 89%, 90%, 91%, 92%, 93%, 94%, 95%, 96%, 97%, 98%, 99%, or more sequence homology with the polypeptide sequence of the present invention when using the method described herein (e.g., BLAST analyses using standard parameters).

In the present invention, a mutant of the amino acid sequence refers to a sequence having more than 70%, for example, more than 75%, 80%, 85%, 90%, 91%, 92%, 93%, 94%, 95%, 96%, 97%, 98%, or 99% homology with the amino acid sequence, e.g., a sequence with three, two, or one substituted, deleted or added amino acids. Preferably, no more than three amino acids are substituted, added or deleted. More preferably, no more than two amino acids are substituted, added or deleted. Most preferably, no more than one amino acid is substituted, added or deleted.

A "substitution" variant is a variant in which at least one amino acid residue in the natural sequence is removed and a different amino acid is inserted in the same position therein. The substitution may be single, wherein only one amino acid in the molecule is substituted; or may be multiple, wherein two or more amino acids in the same molecule are substituted. The multiple substitutions may be at consecutive sites. Similarly, an amino acid can be substituted by multiple residues, wherein such variants comprise both substitutions and insertions. An "insertion" (or "addition") variant is a variant in which one or more amino acids are inserted immediately adjacent to amino acids at a specific position in a natural sequence. Immediately adjacent to an amino acid means linked to either the $\alpha$-carboxyl or $\alpha$-amino functional group of the amino acid. A "deletion" variant is a variant in which one or more amino acids in the natural amino acid sequence are removed. Generally, a deletion variant has one or two amino acids deleted in a specific region of its molecule.

In certain embodiments, fewer than the theoretical maximum number of drug modules are conjugated to the antibody in the conjugation reaction. Generally, the antibody does not contain many free and reactive cysteine thiol groups, which can link drug modules; and in fact, most cysteine thiol groups in the antibody exist as disulfide bridges. In certain embodiments, the antibody can be reduced with a reducing agent such as dithiothreitol (DTT) or tris(2-carboxyethyl) phosphine (TCEP) under partial or complete reducing conditions to produce reactive cysteine thiol groups.

In the present invention, the term "pharmaceutically acceptable salt" refers to (i) salts formed from acidic functional groups present in the conjugate provided by the present invention and appropriate inorganic or organic cations (bases), including but not limited to alkali metal salts, such as sodium salts, potassium salts, and lithium salts; alkaline earth metal salts, such as calcium salts and magnesium salts; other metal salts, such as aluminum salts, iron salts, zinc salts, copper salts, nickel salts, and cobalt salts; inorganic base salts, such as ammonium salts; and organic base salts, such as tert-octylamine salts, dibenzylamine salts, morpholine salts, glucosamine salts, alkyl phenylglycinate salts, ethylenediamine salts, N-methylglucosamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenylethylamine salts, piperazine salts, tetramethylamine salts, and tris(hydroxymethyl)aminomethane salts; and (ii) salts formed from basic functional groups present in the conjugate provided by the present invention and appropriate inorganic or organic anions (acids), including but not limited to hydrohalides, such as hydrofluorides, hydrochlorides, hydrobromides, and hydroiodides; inorganic acid salts, such as nitrates, perchlorates, sulfates, and phosphates; lower alkane sulfonates, such as methanesulfonates, trifluoromethanesulfonates, and ethanesulfonates; arylsulfonates, such as benzenesulfonates and p-benzenesulfonates; organic acid salts, such as acetates, malates, fumarates, succinates, citrates, tartrates, oxalates, and maleates; and amino acid salts, such as glycine salts, trimethylglycine salts, arginine salts, ornithine salts, glutamic acid salts, and aspartic acid salts.

Pharmaceutically acceptable salts can be obtained using standard procedures well known in the art, for example, by reacting a sufficient amount of a basic substance with a suitable acid providing a pharmaceutically acceptable anion, or by reacting a sufficient amount of an acidic substance with a suitable base providing a pharmaceutically acceptable cation.

In the present invention, a solvate refers to the following form of the antibody-drug conjugate of the present invention: a solid or liquid complex formed by coordination of the antibody-drug conjugate with solvent molecules. A hydrate is a specific form of solvate, which has coordinated water molecules. In the present invention, the hydrate is a preferred solvate.

Methods for preparing various pharmaceutical compositions containing certain amounts of active ingredients are known or apparent to those skilled in the art according to the invention of the present invention. As described in REMINGTON' S PHARMACEUTICAL SCIENCES, Martin, E.W., ed., Mack Publishing Company, 19th ed. (1995), a method for preparing the pharmaceutical composition comprises incorporating appropriate pharmaceutical excipients, carriers, diluents, etc., which are non-toxic to cells or mammals exposed thereto at the used dosage and concentration.

In the present invention, pharmaceutical auxiliary materials refer to excipients and additives used during drug production and prescription deployment, and refer to substances that have been reasonably evaluated in terms of safety except active ingredients and are included in pharmaceutical preparation. Pharmaceutical auxiliary materials not only form dosage forms, act as carriers, and enhance stability, but also have important functions such as solubilization, dissolution enhancement, and sustained and controlled release. They are important ingredients that may affect the quality, safety, and efficacy of drug products. According to the sources thereof, they can be divided into natural, semi-synthetic, and fully synthetic. According to the functions and uses thereof, they can be divided into: solvents, propellants, solubilizers, cosolvents, emulsifiers, colorants, binders, disintegrants, fillers, lubricants, wetting agents, osmotic pressure regulators, stabilizers, glidants, correctants, preservatives, suspending agents, coating materials, fragrances, anti-adhesives, antioxidants, chelating agents, penetration enhancers, pH regulators, buffers, plasticizers, surfactants, foaming agents, defoamers, thickeners, inclusion agents, humectants, absorbents, diluents, flocculants and deflocculants, filter aids, release retardants, etc. According to the routes of administration thereof, they can be divided into pharmaceutical auxiliary materials given via oral administration, injection, mucosal administration, transdermal or local administration, nasal or oral inhalation administration, ocular administration, etc. The same pharmaceutical auxiliary material can be used in pharmaceutical preparations for different routes of administration and have different functions and uses.

**In** the present invention, the pharmaceutical composition can be prepared into various suitable dosage forms

according to the route of administration. Examples are tablets, capsules, granules, oral solutions, oral suspensions, oral emulsions, powders, tinctures, syrups, injections, suppositories, ointments, creams, pastes, ophthalmic preparations, pills, implants, aerosols, inhalation powders, sprays, etc. The pharmaceutical composition or suitable dosage form can contain 0.01 mg to 1000 mg of the antibody-drug conjugate, or the pharmaceutically acceptable salt or solvate thereof, or the solvate of the salt of the present invention.

As used herein, the term "treatment" generally refers to obtaining a desired pharmacological and/or physiological effect. The effect may be prophylactic on the basis of the complete or partial prevention of a disease or symptoms thereof; and/or the effect may be therapeutic on the basis of the partial or complete stabilization or cure of the disease and/or side effects due to the disease. As used herein, "treatment" covers any treatment of a disease in a patient, including: (a) prevention of a disease or symptom in a patient who is susceptible to the disease or symptom but has not been diagnosed with the disease; (b) suppressing a symptom of a disease, i.e., preventing the development thereof; or (c) relieving a symptom of a disease, i.e., causing the disease or symptom to resolve.

In the present invention, a "subject" refers to a vertebrate. In certain embodiments, a vertebrate refers to a mammal. Mammals include, but are not limited to, livestock (such as cattle), pets (such as cats, dogs, and horses), primates, mice, and rats. In certain embodiments, a mammal refers to a human.

In the present invention, an "effective amount" refers to an amount effective, at dosages and for periods of time necessary, to achieve a desired therapeutic or prophylactic effect. A "therapeutically effective amount" of the substance/molecule of the present invention may vary depending on factors such as the disease state, age, sex and body weight of the individual and the ability of the substance/molecule to elicit a desired response in the individual. The therapeutically effective amount further covers an amount in which the therapeutically beneficial effect of the substance/molecule outweighs any toxic or harmful consequences. A "prophylactically effective amount" refers to an amount effective, at dosages and for periods of time necessary, to achieve a desired prophylactic effect. Usually but not necessarily, since the prophylactic dose is given to the subject before the onset of the disease or at the early stage of the disease, the prophylactically effective amount will be lower than the therapeutically effective amount. In the case of cancer, the therapeutically effective amount of a drug can reduce the number of cancer cells; reduce the tumor volume; inhibit (i.e., slow down to some extent, preferably stop) the infiltration of cancer cells into surrounding organs; inhibit (i.e., slow down to some extent, preferably stop) tumor metastasis; inhibit tumor growth to some extent; and/or alleviate one or more symptoms related to cancer to some extent.

In the present invention, 20 conventional amino acids and abbreviations thereof follow conventional usages. See Immunology-A Synthesis (2nd edition, E.S. Golub and D.R. Gren, Eds., Sinauer Associates, Sunderland, Mass. (1991)), which is incorporated herein by reference.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to a non-human (e.g., mouse) antibody format, which is a chimeric immunoglobulin, immunoglobulin chain, or a fragment thereof (e.g., Fv, Fab, Fab', F(ab')2, or other antigen-binding subsequences of the antibody) and contains minimal sequence derived from non-human immunoglobulin. Preferably, the humanized antibody is a human immunoglobulin (recipient antibody) in which a residue in the complementarity determining region (CDR) of the recipient antibody is replaced by a CDR residue from a non-human species (donor antibody) such as mouse, rat, or rabbit having the desired specificity, affinity, and capacity.

In addition, during humanization, it is also possible to mutate amino acid residues in CDR1, CDR2 and/or CDR3 regions of VH and/or VL, thereby improving one or more binding properties (e.g., affinity) of the antibody. Mutations can be introduced, for example, by PCR-mediated mutations, the effect of which on antibody binding or other functional properties can be evaluated using *in vitro* or *in vivo* assays as described herein. Generally, conservative mutations are introduced. Such mutations may be amino acid substitutions, additions or deletions. In addition, there are generally no more than one or two mutations within a CDR.

The present invention will be further explained with reference to specific examples below, and these examples do not limit the scope of the present invention.

Example 1. Preparation, sequence, and characterization of humanized GPC3 antibody

I. Preparation of humanized GPC3 antibody

In order to obtain mouse monoclonal antibodies targeting human GPC3, BALB/c mice and C57BL/6 mice were immunized with human GPC3 protein, and the antibody titer in serum was detected by using ELISA method (human GPC3 protein) and FACS method (CHO-K1 cells overexpressing human GPC3). After several rounds of immunization, mice with a higher titer were selected to establish an anti-human GPC3 hybridoma cell line, and positive clones were identified by

means of ELISA method and FACS method. Subsequently, subclones were screened and identified, and monoclonal 52H5D3B8 was obtained. By means of an antibody humanization design, novel humanized antibodies Hu 52H5D3B8-7 and Hu 52H5D3B8-8 were finally obtained.

II. Sequence and characterization of humanized GPC3 antibody

1. Antibody sequence

Hu 52H5D3B8-7:

VH:

EVQLVQSGAEVKKPGASVKVSCKASGYTFTDYEMHWVRQAPGQGLEWMGAIHPGSGGTAYNQKFKGRVTMTADKSISTAYMELSRLRSDDTAVYYCTRFYSYAYWGQGTLVTVSS (SEQ ID NO: 8)

wherein the underlined parts are CDR1 (SEQ ID NO: 1), CDR2 (SEQ ID NO: 2), and CDR3 (SEQ ID NO: 3) in order from left to right; and
the non-underlined parts are FR1 (SEQ ID NO: 4), FR2 (SEQ ID NO: 5), FR3 (SEQ ID NO: 6), and FR4 (SEQ ID NO: 7) in order from left to right.

VL:

DVVMTQTPLSLSVTPGQPASISCRSSQSLVHSNGNTYLQWYLQKPGQSPQLLIYKVSNRFSGVPDRFSGSGSGTDFTLKISRVEAEDVGVYYCSQSIHVPYTFGGGTKVEIK (SEQ ID NO: 16)

wherein the underlined parts are CDR1 (SEQ ID NO: 9), CDR2 (SEQ ID NO: 10), and CDR3 (SEQ ID NO: 11) in order from left to right; and
the non-underlined parts are FR1 (SEQ ID NO: 12), FR2 (SEQ ID NO: 13), FR3 (SEQ ID NO: 14), and FR4 (SEQ ID NO: 15) in order from left to right.

Hu 52H5D3B8-8:

VH:

EVQLVQSGAEVKKPGASVKVSCKASGYTFTDYEMHWVRQAPGQGLEWMGAIHPGSGGTAYNQKFKGRVTMTADKSISTAYMELSRLRSDDTAVYYCTRFYSYAYWGQGTLVTVSS (SEQ ID NO: 8)

wherein the underlined parts are CDR1 (SEQ ID NO: 1), CDR2 (SEQ ID NO: 2), and CDR3 (SEQ ID NO: 3) in order from left to right; and
the non-underlined parts are FR1 (SEQ ID NO: 4), FR2 (SEQ ID NO: 5), FR3 (SEQ ID NO: 6), and FR4 (SEQ ID NO: 7) in order from left to right.

VL:

DVVMTQTPLSLSVTPGQPASISCRSSQSLVHSNGNTYLQWYLQKPGQSPQLLIYKVSNRFSGVPDRFSGSGSGTDFTLKISRVEAEDVGVYFCSQSIHVPYTFGGGTKVEIK (SEQ ID NO: 18)

wherein the underlined parts are CDR1 (SEQ ID NO: 9), CDR2 (SEQ ID NO: 10), and CDR3 (SEQ ID NO: 11) in order from left to right; and
the non-underlined parts are FR1 (SEQ ID NO: 12), FR2 (SEQ ID NO: 13), FR3 (SEQ ID NO: 17), and FR4 (SEQ ID NO: 15) in order from left to right.

The heavy chain constant regions of Hu 52H5D3B8-7 and Hu 52H5D3B8-8 are both:

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO: 19)

The light chain constant regions of Hu 52H5D3B8-7 and Hu 52H5D3B8-8 are both:

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 20)

Control antibody:

(1) GC33 antibody (Chugai Pharmaceutical)

The preparation of the GC33 antibody is described in US 7919086 B2.
The CDR information of the heavy chain and light chain of the GC33 antibody is as follows:

| Antibody | CDR | Amino acid sequence | SEQ ID NO |
|---|---|---|---|
| GC33 (H) | CDR1 | DYEMH | 21 |
| | CDR2 | ALDPKTGDTAYSQKFKG | 22 |
| | CDR3 | FYSYTY | 23 |
| GC33 (L) | CDR1 | RSSQSLVHSNGNTYLH | 24 |
| | CDR2 | KVSNRFS | 25 |
| | CDR3 | SQNTHVPPT | 26 |

The FR information of the heavy chain and light chain of the GC33 antibody is as follows:

| Antibody | FR | Amino acid sequence | SEQ ID NO |
|---|---|---|---|
| GC33 (H) | FR1 | QVQLVQSGAEVKKPGASVKVSCKASGYTFT | 27 |
| | FR2 | WVRQAPGQGLEWMG | 28 |
| | FR3 | RVTLTADKSTSTAYMELSSLTSEDTAVYYCTR | 29 |
| | FR4 | WGQGTLVTVSS | 30 |
| GC33 (L) | FR1 | DVVMTQSPLSLPVTPGEPASISC | 31 |
| | FR2 | WYLQKPGQSPQLLIY | 32 |
| | FR3 | GVPDRFSGSGSGTDFTLKISRVEAEDVGVYYC | 33 |
| | FR4 | FGQGTKLEIK | 34 |

(2) Y035 antibody (CARSGEN Therapeutics)

The preparation of the Y035 antibody is described in CN 106397593 B. The information about the amino acid sequence of the variable regions of the heavy chain and light chain of the humanized Y035 antibody is as follows:

Humanized Y035 heavy chain variable region (SEQ ID NO: 42):

EVQLVQSGAEVKKPGASVKVSCKASGYTHSDYEMHWVRQAPGQGL
EWMGAIHPGSGDTAYNQRFKGRVTITADKSTSTAYMELSSLRSEDTAVYY
CARFYSYAYWGQGTLVTVSA

wherein the underlined parts are CDR1 (SEQ ID NO: 35), CDR2 (SEQ ID NO: 36), and CDR3 (SEQ ID NO: 37) in order from left to right; and
the non-underlined parts are FR1 (SEQ ID NO: 38), FR2 (SEQ ID NO: 39), FR3 (SEQ ID NO: 40), and FR4 (SEQ ID NO: 41) in order from left to right.

Humanized Y035 light chain variable region (SEQ ID NO: 50):

DIVMTQTPLSLPVTPGEPASISCRSSQSLVHSNGNTYLQWYLQWYLQK
PGQSPQLLIYKVSNRFSGVPDRFSGSGSGTDFTLKISRVEAEDVGVYYCSQS
IYVPYTFGQGTKLEIKR

wherein the underlined parts are CDR1 (SEQ ID NO: 43), CDR2 (SEQ ID NO: 44), and CDR3 (SEQ ID NO: 45) in order from left to right; and
the non-underlined parts are FR1 (SEQ ID NO: 46), FR2 (SEQ ID NO: 47), FR3 (SEQ ID NO: 48), and FR4 (SEQ ID NO: 49) in order from left to right.

The heavy chain constant regions of the GC33 antibody and the Y035 antibody are both:

ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTC LVKGFYPSDIAVEWESNGQPENNYKTTPPVLDSDGSFFLYSKLTVDKSRW QQGNVFSCSVMHEALHNHYTQKSLSLSPGK (SEQ ID NO: 19)

The light chain constant regions of the GC33 antibody and the Y035 antibody are both:

RTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPV TKSFNRGEC (SEQ ID NO: 20)

2. Binding of humanized GPC3 antibody to cells

The inventors evaluated the binding activities of Hu 52H5D3B8-7 and Hu 52H5D3B8-8 to CHO-K1 cells, HepG2 cells, and HuH-7 cells overexpressing human GPC3 by FACS, and made a comparison with the known anti-GPC3 antibodies GC33 and Y035. In order to evaluate the CDR regions of the two antibodies Hu 52H5D3B8-7 and Hu 52H5D3B8-8 and the FR regions of the antibody Hu 52H5D3B8-7, the inventors prepared two recombinant antibodies. "Hu 52H5D3B8-7/8 CDRs with Y035 FRs" means that the antibody is formed by recombining the CDR regions of the antibody Hu 52H5D3B8-7 or Hu 52H5D3B8-8 with the FR regions of the Y035 antibody. The antibody "Y035 CDRs with Hu 52H5D3B8-7 FRs" means that the antibody is formed by recombining the CDR regions of the Y035 antibody with the FR regions of the antibody Hu 52H5D3B8-7.

Firstly, the GPC3-CHO-K1 cells, HepG2 cells, and Huh-7 cells were incubated with an Fc blocker at 4°C for 15 minutes. The cells were washed with FACS buffer (PBS + 2% FBS), and 5-fold serially diluted solutions of Hu 52H5D3B8-7, Hu 52H5D3B8-8, Hu 52H5D3B8-7/8 CDRs with Y035 FRs, Y035 CDRs with Hu 52H5D3B8-7 FRs, or Y035 mAb (starting concentration 100 nM) were added to each well and incubated at 4°C for 60 minutes. The cells were washed with FACS buffer and fixed with 2% PFA for 15 minutes at room temperature. After washing with FACS buffer, Alexa Fluor® 647 AffiniPure goat anti-human IgG was added to each well and incubated at room temperature for 30 minutes. The sample was washed twice with FACS buffer. The cell sample was analyzed for fluorescence signal by flow cytometer MACSQuant Analyzer 16 and expressed by MFI (GeoMean fluorescence intensity). The experimental results are as shown in Figures 1-3, in which Hu 52H5D3B8-7 and Hu 52H5D3B8-8 have comparable binding affinities for the GPC3-CHO-K1 cells, HepG2 cells, and Huh-7 cells, which are higher than the other antibodies. In addition, both the antibody "Hu 52H5D3B8-7/8 CDRs with Y035 FRs" and the antibody "Y035 CDRs with Hu 52H5D3B8-7 FR" exhibit higher cell affinities than Y035, which means that the CDR regions of the antibodies Hu 52H5D3B8-7 and Hu 52H5D3B8-8 or the FR regions of the antibody Hu 52H5D3B8-7 are both superior to those of the Y035 antibody. The $EC_{50}$ values of each test antibody on the GPC3-CHO-K1 cells, HepG2 cells, and Huh-7 cells are as shown in Table 1 below.

Table 1: Affinities of each GPC3 antibody for GPC3-CHO-K1 cells, HepG2 cells, and Huh-7 cells ($EC_{50}$)

| Antibody name | $EC_{50}$ (nM) | | |
|---|---|---|---|
| | GPC3-CHO-K 1 | HepG2 | Huh-7 |
| Hu 52H5D3B8-7 | 0.4459 | 0.4051 | 0.4637 |
| Hu 52H5D3B8-8 | 0.4106 | 0.3512 | 0.4177 |
| GC33 | 0.9556 | 0.7449 | 0.9528 |
| Y035 | 1.460 | 1.250 | 1.178 |
| Hu 52H5D3B8-7/8 CDRs with Y035 FRs | 0.7627 | 0.7687 | 0.7824 |

(continued)

| Antibody name | $EC_{50}$ (nM) | | |
|---|---|---|---|
| | GPC3-CHO-K 1 | HepG2 | Huh-7 |
| Y035 CDRs with Hu 52H5D3B8-7 FRs | 0.9072 | 0.6107 | 0.6674 |

3. Cellular internalization of humanized GPC3 antibody

The internalization of Hu 52H5D3B8-7 and Hu 52H5D3B8-8 on the HepG2 cells was evaluated and compared with the known GPC3 antibody Y035 (CARSGEN Therapeutics) for internalization. In addition, in order to evaluate the CDRs and FRs of the antibodies Hu 52H5D3B8-7 and Hu 52H5D3B8-8, the inventors also evaluated the antibody "Hu 52H5D3B8-7/8 CDRs with Y035 FRs" and the antibody "Y035 CDRs with Hu 52H5D3B8-7 FRs" in an *in vitro* internalization experiment.

pHAb thiol dye is a pH sensor dye, which has very low fluorescence at pH > 7 and significantly increased fluorescence when it is internalized into endosomes or lysosomes (at pH 6.3 or 4.7, respectively). In brief, α-hIgG secondary antibody labeled with the pHAb thiol dye (10 μg/mL) was incubated with the antibody (20 μg/mL) for 30 minutes. After incubation, the mixed antibody was 2-fold serially diluted, and the diluted mixture was added to each well in a 96-well assay plate pre-seeded with $2 \times 10^4$ HepG2 cells. After 48 hours of culture, the fluorescence signal was captured on a multimode reader (Envision® 2105). The experimental results are as shown in Figure 4, in which both Hu 52H5D3B8-7 and Hu 52H5D3B8-8 show higher internalization effects than the other antibodies. In addition, the antibody "Hu 52H5D3B8-7/8 CDRs with Y035 FRs" shows a higher internalization effect (a higher Top value and comparable $EC_{50}$) than Y035, which means that the CDRs of Hu 52H5D3B8-7 and Hu 52H5D3B8-8 are superior to those of Y035. The $EC_{50}$ and Top values of each test antibody are as shown in Table 2 below.

Table 2: Endocytosis ($EC_{50}$, Top) of each GPC3 antibody on HepG2 cells

| Antibody name | $EC_{50}$ (μg/mL) | Top |
|---|---|---|
| Hu 52H5D3B8-7 | 0.7195 | 58555 |
| Hu 52H5D3B8-8 | 0.7805 | 62378 |
| Y035 | 1.078 | 51973 |
| Hu 52H5D3B8-7/8 CDRs with Y035 FRs | 1.142 | 59223 |
| Y035 CDRs with Hu 52H5D3B8-7 FRs | 1.107 | 52157 |

Example 2. Preparation of GPC3-ADC

1. Preparation of antibody-drug conjugate Anti-GPC3-L1-D1

a: 10 mg of GPC3 antibody (Hu 52H5D3B8-7, Hu 52H5D3B8-8, or GC33) was taken, and the pH of the antibody was adjusted to 7.5 with a 0.7 M Tris-0.5 M EDTA solution, followed by weighing, protein concentration detection, and total protein amount calculation. 2.8 folds by number of moles of TCEP was added to the antibody, and the mixture was placed on a 3D shaker and reacted at a temperature of 22°C for 105 minutes with continuous mixing.

b: L1-D1, i.e., MC-vc-PAB-MMAE (produced by Waterstone Pharmaceuticals (Hubei) Co., Ltd. entrusted by Shanghai Miracogen Inc., dissolved in DMSO), was added to the reduced antibody in an amount of 1.6 folds of the number of moles of free thiol groups. After uniform mixing, the mixture was placed on a 3D shaker and reacted at a temperature of 22°C for 20 minutes with continuous mixing. N-acetylcysteine was added to the reaction system in an amount of 3 folds of the number of moles of L1-D1 (i.e., MC-vc-PAB-MMAE) added in the reaction solution. After uniform mixing, the mixture was placed on a 3D shaker and reacted at a temperature of 22°C for 5 minutes with continuous mixing. After a total of five instances of exchange into a conjugate storage solution (10 mM histidine, 3% sucrose, and 0.03% Tween-80, pH 5.6) by using a 15 mL 30 KD ultrafiltration device, filtration was carried out by using a 0.22 μm filter membrane to obtain the antibody-drug conjugate product Anti-GPC3-L1-D1 (a general term of Hu 52H5D3B8-7-L1-D1, Hu 52H5D3B8-8-L1-D1, and GC33-L1-D1), which was stored at 4°C.

2. Preparation of antibody-drug conjugate Anti-GPC3-L3-D3

a: 2 mg of GPC3 antibody (Hu 52H5D3B8-7, Hu 52H5D3B8-8, or GC33) was taken, and the pH of the antibody was adjusted to 7.5 with a 0.7 M Tris-0.5 M EDTA solution, followed by weighing, protein concentration detection, and total protein amount calculation. 1.3 folds by number of moles of TCEP was added to the antibody, and the mixture was placed on a 3D shaker and reacted at a temperature of 22°C for 105 minutes with continuous mixing.
b: L3-D3, i.e., MA-PEG4-VC-PAB-DMEA-Duocarmycin SA (purchased from Levena (Suzhou) Biopharma Co., Ltd., dissolved in DMSO), was added to the reduced antibody in an amount of 2 folds of the number of moles of free thiol groups. After uniform mixing, the mixture was placed on a 3D shaker and reacted at a temperature of 22°C for 20 minutes with continuous mixing. N-acetylcysteine was added to the reaction system in an amount of 3 folds of the number of moles of L3-D3 (i.e., MA-PEG4-VC-PAB-DMEA-Duocarmycin SA) added in the reaction solution. After uniform mixing, the mixture was placed on a 3D shaker and reacted at a temperature of 22°C for 5 minutes with continuous mixing. After a total of five instances of exchange into a conjugate storage solution (10 mM histidine, 3% sucrose, and 0.03% Tween-80, pH 5.6) by using a 0.5 mL 30 KD ultrafiltration device, filtration was carried out by using a 0.22 μm filter membrane to obtain the antibody-drug conjugate product Anti-GPC3-L3-D3 (a general term of Hu 52H5D3B8-7-L3-D3, Hu 52H5D3B8-8-L3-D3, and GC33-L3-D3), which was stored at 4°C.

3. Preparation of antibody-drug conjugate Anti-GPC3-L4-D4

a: 10 mg of GPC3 antibody (Hu 52H5D3B8-7, Hu 52H5D3B8-8, or GC33) was taken, and the pH of the antibody was adjusted to 7.5 with a 0.7 M Tris-0.5 M EDTA solution, followed by weighing, protein concentration detection, and total protein amount calculation. 2.7 folds by number of moles of TCEP was added to the antibody, and the mixture was placed on a 3D shaker and reacted at a temperature of 22°C for 105 minutes with continuous mixing.
b: L4-D4, i.e., MC-MMAF (purchased from BrightGene Bio-Medical Technology (Suzhou) Co., Ltd., dissolved in DMSO), was added to the reduced antibody in an amount of 2.0 folds of the number of moles of free thiol groups. After uniform mixing, the mixture was placed on a 3D shaker and reacted at a temperature of 22°C for 20 minutes with continuous mixing. N-acetylcysteine was added to the reaction system in an amount of 3 folds of the number of moles of L4-D4 (i.e., MC-MMAF) added in the reaction solution. After uniform mixing, the mixture was placed on a 3D shaker and reacted at a temperature of 22°C for 5 minutes with continuous mixing. After a total of five instances of exchange into a conjugate storage solution (10 mM histidine, 3% sucrose, and 0.03% Tween-80, pH 5.6) by using a 15 mL 30 KD ultrafiltration tube, filtration was carried out by using a 0.22 μm filter membrane to obtain the antibody-drug conjugate product Anti-GPC3-L4-D4 (a general term of Hu 52H5D3B8-7-L4-D4, Hu 52H5D3B8-8-L4-D4, and GC33-L4-D4), which was stored at 4°C.

4. Preparation of antibody-drug conjugate Anti-GPC3-ZLA

a: 10 mg of GPC3 antibody (Hu 52H5D3B8-7, Hu 52H5D3B8-8, or GC33) was taken, and the antibody was buffer-exchanged into 40 mM PB + 2 mM EDTA (pH 7.0). The antibody was diluted into a concentration of 5 mg/mL with a 40 mM PB + 2 mM EDTA (pH 6.99) solution, followed by weighing, protein concentration detection, and total protein amount calculation. 2.25 folds by number of moles of TCEP was added to the antibody, and the sample was uniformly mixed, placed in a constant-temperature incubator, and reacted at a temperature of 22°C for 3 hours with continuous mixing, with the speed of the mixer being 200 rpm.
b: The reduced antibody solution was cooled on ice, and ZLA (dissolved in DMA, with the total content of DMA being 10% in the reaction system; ZLA is the compound shown in Example 2.1 disclosed in CN 107001415 B, with the structural formula being as shown below) was added to the cooled antibody in an amount of 7 folds of the number of moles of the antibody. After rapid uniform mixing, the mixture was placed in a constant-temperature incubator and reacted at a temperature of 4°C for 2 hours with continuous mixing, with the speed of the mixer being 200 rpm. N-acetylcysteine was added to the reaction system in an amount of 6 folds of the number of moles of the antibody added in the reaction solution. After uniform mixing, the mixture was placed in a constant-temperature incubator, and reacted at a temperature of 22°C for 15 minutes with continuous mixing, with the speed of the mixer being 200 rpm. After exchange into a conjugate storage solution (20 mM histidine/histidine-HCl, pH 5.41) by using a 15 mL 50 KD ultrafiltration tube, sterilization filtration was carried out by using a 0.22 μm filter membrane, followed by protein

concentration detection. The antibody-drug conjugate product Anti-GPC3-ZLA (a general term of Hu 52H5D3B8-7-ZLA, Hu 52H5D3B8-8-ZLA, and GC33-ZLA) was obtained and stored at 4°C.

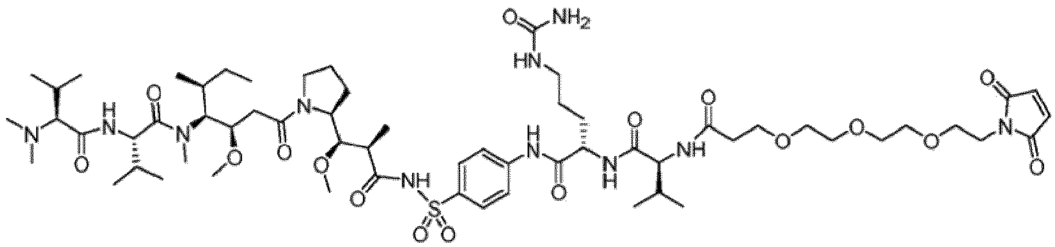

Structural formula of ZLA

The drug-to-antibody ratio (DAR) of the above GPC3-ADCs was detected by using hydrophobic interaction chromatography (HIC-HPLC). Their typical chromatograms are as shown in Figures 5-8. The average drug-to-antibody ratios (DARs) of Hu 52H5D3B8-7-L1-D1, Hu 52H5D3B8-7-L3-D3, Hu 52H5D3B8-7-L4-D4, and Hu 52H5D3B8-8-ZLA were 4.2, 2.3, 4.0, and 3.9, respectively, as calculated according to the peak area in the chromatogram.

Example 3. Pharmacological and pharmacodynamic study of GPC3-ADC

1. Binding of GPC3-ADC to cells

It was confirmed by an FACS experiment that the binding of ADC was basically unaffected by Cys conjugation, and at the same time Hu 52H5D3B8-8 ADC and GC33 ADC were compared for cell binding.

After digestion by trypsin-EDTA, the cultured HepG2 cells were collected. After centrifugation, the cells were resuspended in FACS buffer (PBS + 2% FBS), the cell density was adjusted, and the cells were dispensed to a 96-well U-bottom cell culture plate, such that each well contained 200,000-500,000 cells. The antibody or ADC diluted with FACS buffer in a 4-fold gradient manner was added and uniformly mixed, such that the final starting concentration of each sample was finally 10 μg/mL. The 96-well plate was incubated at 4°C for 60 minutes. The cells in the wells were thoroughly washed with FACS buffer to remove unbound antibodies and ADCs. Goat anti-Human IgG (H+L) Secondary Antibody, Alexa Fluor 488 (Invitrogen), diluted with FACS buffer at 1 : 1,000 was added and incubated at 4°C in the dark for 30 minutes. The cells in the wells were washed with FACS buffer to remove unbound secondary antibodies. The cell sample was analyzed for fluorescence signal by CytoFLEX flow cytometer (Beckman Coulter). The experimental results are as shown in Figure 9, in which the affinities of the antibodies Hu 52H5D3B8-8 and GC33 for the HepG2 cells are not changed before and after conjugation with the cytotoxin, and the affinities of the antibody Hu 52H5D3B8-8 and Hu 52H5D3B8-8 ADC for the HepG2 cells are stronger than those of the GC33 antibody and GC33 ADC. The affinity of each test substance for the HepG2 cells is as shown in Table 3.

Table 3: Binding ($EC_{50}$) of each GPC3 antibody and ADC to HepG2 cells

| Antibody/ADC name | $EC_{50}$ (ng/mL) | Top |
|---|---|---|
| Hu 52H5D3B8-8 | 74.31 | 75037 |
| Hu 52H5D3B8-8-L4-D4 | 69.29 | 72883 |
| Hu 52H5D3B8-8-ZLA | 56.90 | 69119 |
| GC33 | 268.8 | 64119 |
| GC33-L4-D4 | 111.9 | 60204 |
| GC33-ZLA | 142.7 | 68043 |

2. Internalization of antibody in GPC3-ADC

It was confirmed that the internalization of ADC was not weaker than that of the monoclonal antibody, and at the same time Hu 52H5D3B8-7ADC, Hu 52H5D3B8-8 ADC, and GC33 ADC were compared for internalization.

A 96-well U-bottom cell culture plate was blocked with 5% skimmed milk powder dissolved in PBS for 60 minutes. After being treated with a non-trypsin digestive solution (Sigma-Aldrich), the cultured Huh7 liver cancer cells were collected. After centrifugation, the cells were resuspended in a pre-cooled DMEM medium, the cell density was adjusted,

and the cells were dispensed to a blocked 96-well U-bottom cell culture plate, such that each well contained 500,000-800,000 cells. The antibody and ADC diluted with DMEM medium containing 5% FBS were added and uniformly mixed, such that the final concentration of the sample was 10 μg/mL. The 96-well plate was incubated on ice for 60 minutes. The cells in the wells were thoroughly washed with pre-cooled FACS buffer (PBS + 2% FBS) to remove unbound antibodies and ADCs. The cells in the wells were evenly dispensed to two blocked 96-well U-bottom plates. After centrifugation, the cells were resuspended with DMEM medium. One 96-well plate was placed on ice, and the other was placed in a cell incubator at 37°C. After 2 hours, the 37°C culture plate was transferred to ice and left to stand for 5-10 minutes. The diluted Goat anti-Human IgG (H+L) Secondary Antibody, Alexa Fluor 488 (Invitrogen) was added and incubated on ice in the dark for 30 minutes. The cells in the wells were washed with pre-cooled FACS buffer to remove unbound secondary antibodies. The cell sample was analyzed for fluorescence signal by CytoFLEX flow cytometer (Beckman Coulter) and expressed by MFI (GeoMean fluorescence intensity). Except for incubation at 37°C, the whole process of the experiment was kept on ice or at 4°C.

The calculation method for the percentage reduction of molecules on the cell surface was as follows:

$$\%\ \text{reduction of molecules on cell surface} = (MFI_{\text{on ice}} - MFI_{37^\circ C}) / MFI_{\text{on ice}} * 100\%.$$

The experimental results are as shown in Table 4, in which the internalization abilities of the antibodies Hu 52H5D3B8-8 and GC33 on the Huh7 cells are not changed before and after conjugation with the cytotoxin, and the internalization abilities of the antibody Hu 52H5D3B8-8 and Hu 52H5D3B8-8 ADC are stronger than those of the GC33 antibody and GC33 ADC. The results of the endocytosis of each test substance on the Huh7 cells are as shown in Table 4.

Table 4: Endocytosis of each GPC3 antibody and ADC on Huh7 cells

| Antibody/ADC name | Percentage reduction of GPC3 on cell surface (%) |
|---|---|
| Hu 52H5D3B8-8 | 65.3 |
| Hu 52H5D3B8-8-L1-D1 | 66.5 |
| Hu 52H5D3B8-8-L3-D3 | 65.0 |
| Hu 52H5D3B8-8-L4-D4 | 63.4 |
| Hu 52H5D3B8-8-ZLA | 64.3 |
| GC33 | 60.0 |
| GC33-L1-D1 | 63.1 |
| GC33-L3-D3 | 59.9 |
| GC33-L4-D4 | 61.3 |
| GC33-ZLA | 59.0 |
| Hu 52H5D3B8-7 | 64.4 |
| Hu 52H5D3B8-7-L1-D1 | 70.8 |

Example 4. Pharmacodynamic study *in vitro*

Hu 52H5D3B8-7 or Hu 52H5D3B8-8 ADC and GC33 ADC, to which a variety of linker-payloads (i.e., L-Ds) were conjugated, were compared for cytotoxicity.

After digestion by trypsin-EDTA, the cultured liver cancer cells were collected. After centrifugation, the cells were resuspended in a new medium, and the cells were counted. The cells were inoculated into a 96-well, clear-bottom, black cell culture plate (Costar) and cultured overnight in a cell incubator. The next day, the antibody and ADC were diluted in a 4-fold gradient manner with a medium, and the diluted solution was carefully transferred to the black culture plate such that the final starting concentration of each sample was finally 10 μg/mL. After culturing in a cell incubator for 96 hours, PrestoBlue reagent (Invitrogen) was added to take up 1/10 of the well volume, followd by incubation in a cell incubator for 1 hour. The fluorescence signal was read by SpectraMax M5 plate reader, wherein the excitation and emission wavelengths of the instrument were set to 560 nm and 590 nm, respectively. The obtained fluorescence signal data were analyzed by SoftMax Pro 6.5 software.

1. Experimental reagents and sources:

Test drug

| Name | Preservation condition |
|---|---|
| Hu 52H5D3B8-8-L1-D1 | 2-8°C |
| GC33-L1-D1 | 2-8°C |
| Hu 52H5D3B8-8-L3-D3 | 2-8°C |
| GC33-L3-D3 | 2-8°C |
| Hu 52H5D3B8-7-L4-D4 | 2-8°C |
| GC33-L4-D4 | 2-8°C |
| Hu 52H5D3B8-8-ZLA | 2-8°C |
| GC33-ZLA | 2-8°C |

Cell line

| Cell line | Cell type | GPC3 expression level | Source | Culture solution |
|---|---|---|---|---|
| Huh7 | Human liver cancer cells | Overexpression ($9 * 10^3$ to $1.25 * 10^5$) | Chinese Academy of Sciences | DMEM + 10% FBS |
| HepG2 | Human liver cancer cells | Overexpression ($2.5 * 10^3$ to $9.67 * 10^5$) | ATCC | MEM + 10% FBS |

2. Experimental results:

The average $EC_{50}$ of cytotoxicity of each GPC3-ADC is as shown in Table 5. Figures 10-15 are representative diagrams of the killing effects of various GPC3-ADCs on the Huh7/HepG2 cells.

Table 5: $EC_{50}$ values of cytotoxicity of various GPC3-ADCs in screened cell lines

| Test substance | $EC_{50}$ value (ng/mL) | |
|---|---|---|
| | Huh7 | HepG2 |
| Hu 52H5D3B8-8-L1-D1 | 38.02 | 40.89 |
| GC33-L1-D1 | 78.60 | 73.18 |
| Hu 52H5D3B8-8-L3-D3 | 27.40 | - |
| GC33-L3-D3 | 69.37 | - |
| Hu 52H5D3B8-8-ZLA | 3.52 | 39.14 |
| GC33-ZLA | 14.75 | 89.25 |
| Hu 52H5D3B8-7-L4-D4 | 4.547 | - |
| GC33-L4-D4 | 8.822 | - |
| Notes: "-" represents not detected. | | |

As can be seen from the results in Table 5 and Figures 10-15, the various GPC3-ADCs of the present invention all exhibit significant cell killing activity in cell lines with medium to high expression of GPC3 and are more potent than GC33-ADC.

Example 5. Pharmacodynamic study *in vivo*

The antitumor activity of GPC3-ADCs was tested in an LIV#219 liver cancer PDX model. The model has a higher expression level of GPC3 RNA, and a positive expression of GPC3 as confirmed by FACS analysis.

The process of the establishment of a PDX model of nude mice with human liver cancer was as follows: A tumor tissue with a volume size of about 15-30 $mm^3$ was transplanted subcutaneously under the back of the BALB/c nude mice. When the tumor volume reached 250-360 $mm^3$, the mice were divided into a total of 4 groups by a randomized block method, with 6 mice per group, ensuring uniformity in tumor volume and balance in body weight between these groups. The groups included a vehicle group, a 3 mg/kg GC33-L1-D1 administration group, a 3 mg/kg Hu 52H5D3B8-7-L1-D1

administration group, and a 3 mg/kg Hu 52H5D3B8-8-L1-D1 administration group.

Data analysis: During the experiment, the tumor volume was measured twice a week. The calculation formula for the tumor volume (TV) is: TV = $l \times w^2 / 2$, in which l and w represent the measured length and width of the tumor, respectively. According to the measurement results, the relative tumor volume (RTV) was calculated by RTV = $V_f$ / V0, in which V0 is the tumor volume measured at the time of grouping for administration (i.e., Day 0), and $V_f$ is the tumor volume measured on the last day. Relative tumor proliferation rate (T/C) (%) = (RTV in drug administration group / RTV in vehicle group) × 100%. Tumor growth inhibition rate (TGI %) = (average tumor volume in vehicle group - average tumor volume in drug administration group) / average tumor volume in vehicle group × 100%. When T/C (%) ≤ 40% and $P < 0.05$, the test sample was believed to have a significant inhibitory effect on tumor growth.

The experimental results are as shown in Figures 16 and 17. After administration with GC33-L1-D1 at a dose of 3 mg/kg, on Day 26, the relative tumor proliferation rate (T/C) (%) was 54.90%, and the tumor growth inhibition rate (TGI %) was 45.10%; the Hu 52H5D3B8-7-L1-D1 (3 mg/kg) group had a T/C (%) of 33.58% and a TGI % of 66.42%; and the Hu 52H5D3B8-8-L1-D1 (3 mg/kg) group had a T/C (%) of 39.93% and a TGI % of 60.07%.

The experimental results indicate that Hu 52H5D3B8-7-L1-D1 (3 mg/kg) and Hu 52H5D3B8-8-L1-D1 (3 mg/kg) both have significant inhibitory activities on tumor growth and are superior to GC33-L1-D1 (3 mg/kg). The tumor-bearing mice are well tolerated to both Hu 52H5D3B8-7-L1-D1 and Hu 52H5D3B8-8-L1-D1.

## Claims

1. An antibody-drug conjugate, or a pharmaceutically acceptable salt or solvate thereof, or a solvate of the salt, **characterized in that** the antibody-drug conjugate has a structure represented by formula I,

   $Ab\text{-}(L\text{-}D)_p$ formula I

   in which:

   Ab is an anti-GPC3 antibody, and the anti-GPC3 antibody comprises a heavy chain and a light chain, wherein CDR1, CDR2 and CDR3 in a heavy chain variable region comprise the sequences as set forth in SEQ ID NO: 1, SEQ ID NO: 2 and SEQ ID NO: 3, respectively, and CDR1, CDR2 and CDR3 in a light chain variable region comprise the sequences as set forth in SEQ ID NO: 9, SEQ ID NO: 10 and SEQ ID NO: 11, respectively;
   L is a linker;
   D is a cytotoxin; and
   p is any numerical value between 1 and 8.

2. The antibody-drug conjugate, or the pharmaceutically acceptable salt or solvate thereof, or the solvate of the salt according to claim 1, **characterized in that** the linker is selected from:

   6-maleimidocaproyl (MC), maleimidopropionyl (MP), N-succinimidyl 4-(2-pyridylthio)pentanoate (SPP), 4-(N-maleimidomethyl)-cyclohexane-1-formyl (MCC), N-succinimidyl (4-iodo-acetyl)aminobenzoate (SIAB), 6-maleimidocaproyl-valine-citrulline-p-aminobenzyloxycarbonyl (MC-vc-PAB), MA-PEG4-VC-PAB-DMEA, and

   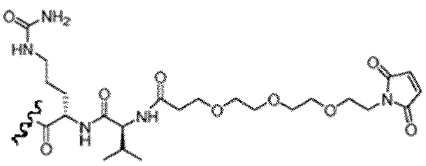

   ;

   preferably, the linker is selected from:
   6-maleimidocaproyl (MC), 6-maleimidocaproyl-valine-citrulline-p-aminobenzyloxycarbonyl (MC-vc-PAB), MA-PEG4-VC-PAB-DMEA, and

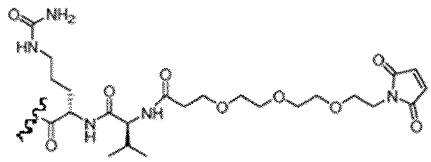

.

3. The antibody-drug conjugate, or the pharmaceutically acceptable salt or solvate thereof, or the solvate of the salt according to either claim 1 or 2, **characterized in that** the cytotoxin is selected from:

SN-38, gemcitabine, monomethyl auristatin E (MMAE), monomethyl auristatin F (MMAF), maytansinoids (e.g., maytansine DM1 and maytansine DM4), calicheamicin, MGBAs (e.g., duocarmycin), doxorubicin, ricin, diphtherin, duocarmycin SA,

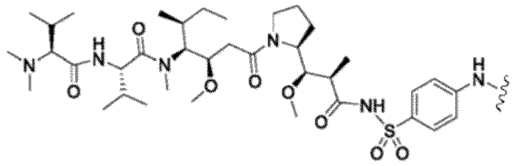

,

I131, interleukins, tumor necrosis factors, chemokines, and nanoparticles;
preferably, the cytotoxin is selected from:
monomethyl auristatin E (MMAE), duocarmycin SA, monomethyl auristatin F (MMAF), and

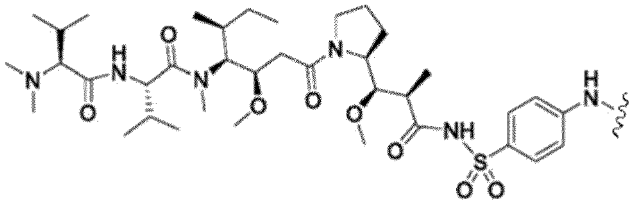

.

4. The antibody-drug conjugate, or the pharmaceutically acceptable salt or solvate thereof, or the solvate of the salt according to any one of claims 1-3, **characterized in that** L-D is selected from:
MC-vc-PAB-MMAE, MA-PEG4-VC-PAB-DMEA-Duocarmycin SA, MC-MMAF, and

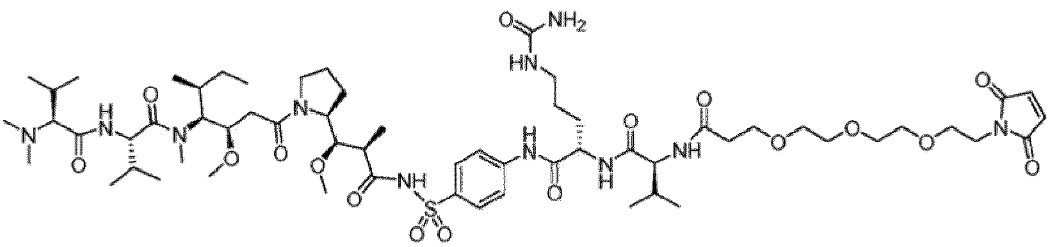

.

5. The antibody-drug conjugate, or the pharmaceutically acceptable salt or solvate thereof, or the solvate of the salt according to any one of claims 1-4, **characterized in that** the anti-GPC3 antibody has one or both of the following features 1) and 2):

1) FR1, FR2, FR3, and FR4 regions in a heavy chain variable region of the anti-GPC3 antibody comprise the sequences as set forth in SEQ ID NO: 4, SEQ ID NO: 5, SEQ ID NO: 6, and SEQ ID NO: 7, respectively; and
2) FR1, FR2, FR3, and FR4 regions in a light chain variable region of the anti-GPC3 antibody comprise the sequences as set forth in SEQ ID NO: 12, SEQ ID NO: 13, SEQ ID NO: 14, and SEQ ID NO: 15, respectively, or FR1, FR2, FR3, and FR4 regions in a light chain variable region of the anti-GPC3 antibody comprise the sequences as set forth in SEQ ID NO: 12, SEQ ID NO: 13, SEQ ID NO: 17, and SEQ ID NO: 15, respectively.

**6.** The antibody-drug conjugate, or the pharmaceutically acceptable salt or solvate thereof, or the solvate of the salt according to any one of claims 1-5, **characterized in that** the anti-GPC3 antibody has one or both of the following features 1) and 2):

1) the sequence of the heavy chain variable region of the anti-GPC3 antibody comprises the sequence as set forth in SEQ ID NO: 8; and
2) the sequence of the light chain variable region of the anti-GPC3 antibody comprises the sequence as set forth in SEQ ID NO: 16 or SEQ ID NO: 18.

**7.** The antibody-drug conjugate, or the pharmaceutically acceptable salt or solvate thereof, or the solvate of the salt according to any one of claims 1-6, **characterized in that** the anti-GPC3 antibody has one or both of the following features 1) and 2):

1) a heavy chain constant region of the anti-GPC3 antibody is selected from human IgG, IgM, IgA, IgD, and IgE constant regions, or mutants of the constant regions;
preferably, the IgG is selected from IgG1, IgG2, IgG3, and IgG4; and
2) a light chain constant region of the anti-GPC3 antibody is human lambda constant region or kappa constant region, or a mutant of the constant region.

**8.** The antibody-drug conjugate, or the pharmaceutically acceptable salt or solvate thereof, or the solvate of the salt according to any one of claims 1-7, **characterized in that** the anti-GPC3 antibody has one or both of the following features 1) and 2):

1) the sequence of the heavy chain constant region of the anti-GPC3 antibody comprises the sequence as set forth in SEQ ID NO: 19, or a sequence with more than 70%, preferably more than 75%, 80%, 85%, 90%, 95%, or 99% homology with the sequence as set forth in SEQ ID NO: 19;
preferably, the sequence of the heavy chain constant region of the anti-GPC3 antibody is as set forth in SEQ ID NO: 19; and
2) the sequence of the light chain constant region of the anti-GPC3 antibody comprises the sequence as set forth in SEQ ID NO: 20, or a sequence with more than 70%, preferably more than 75%, 80%, 85%, 90%, 95%, or 99% homology with the sequence as set forth in SEQ ID NO: 20;

preferably, the sequence of the light chain constant region of the anti-GPC3 antibody is as set forth in SEQ ID NO: 20.

**9.** The antibody-drug conjugate, or the pharmaceutically acceptable salt or solvate thereof, or the solvate of the salt according to any one of claims 1-8, **characterized in that** p is any numerical value between 2 and 8;

preferably, p is any numerical value between 2 and 6;
more preferably, p is any numerical value between 2 and 5 (e.g., 2.3, 3.9, 4.0, or 4.2).

**10.** A pharmaceutical composition **characterized by** comprising the antibody-drug conjugate, or the pharmaceutically acceptable salt or solvate thereof, or the solvate of the salt according to any one of claims 1-9;

optionally further comprising at least one of a chemotherapeutic drug, an immunotherapeutic drug, and an immunosuppressant for treating a tumor;
or optionally further comprising at least one pharmaceutical auxiliary material.

**11.** Use of the antibody-drug conjugate, or the pharmaceutically acceptable salt or solvate thereof, or the solvate of the salt according to any one of claims 1-9 or the pharmaceutical composition according to claim 10 in the preparation of a drug for preventing and/or treating a GPC3 positivity-associated disease, **characterized in that**
preferably, the GPC3 positivity-associated disease includes liver cancer, lung cancer, gastric cancer, head and neck cancer, esophageal cancer, Merkel cell cancer, or liposarcoma.

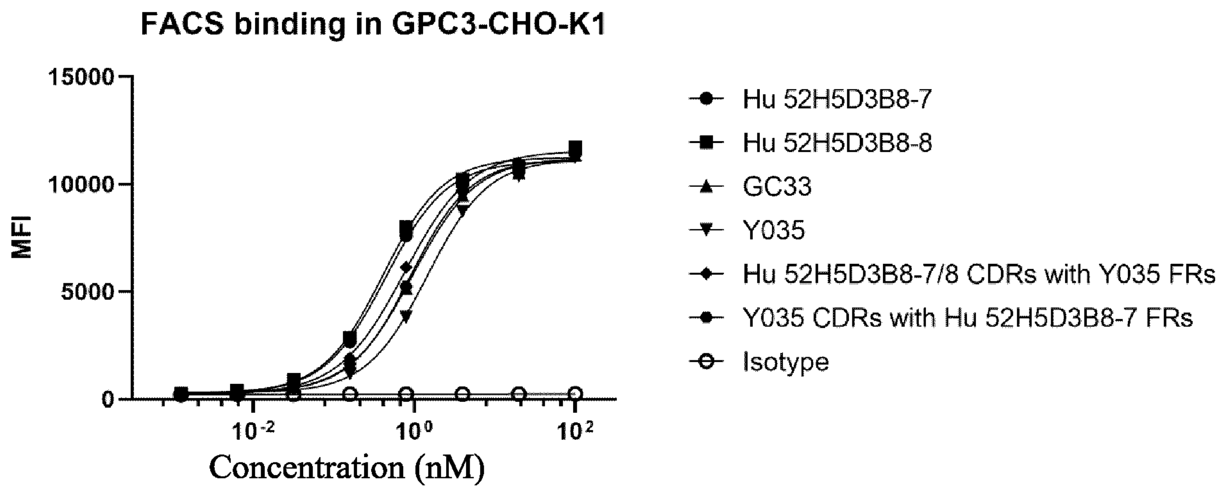


*FIG. 1*

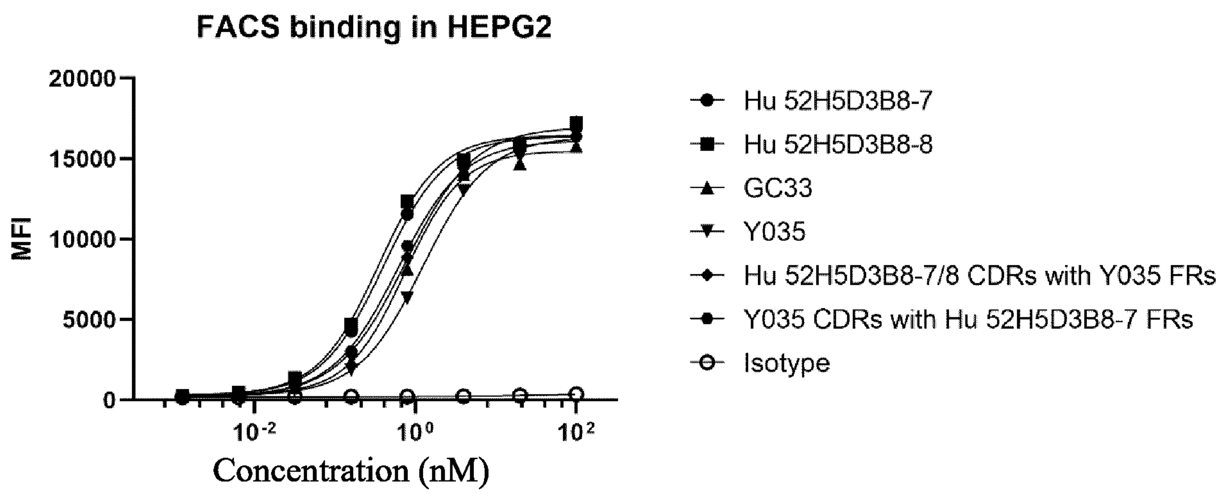


*FIG. 2*

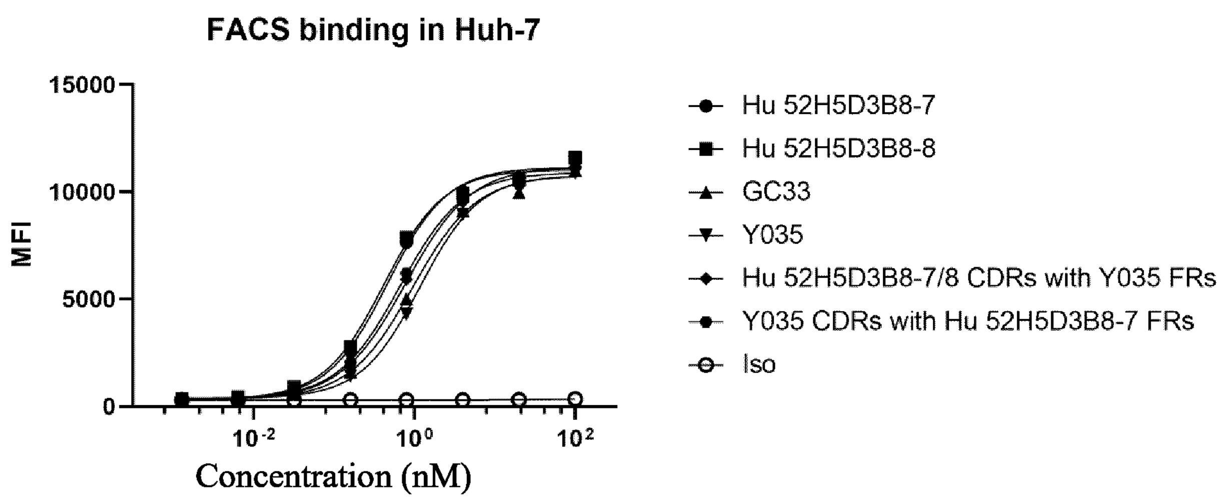


*FIG. 3*

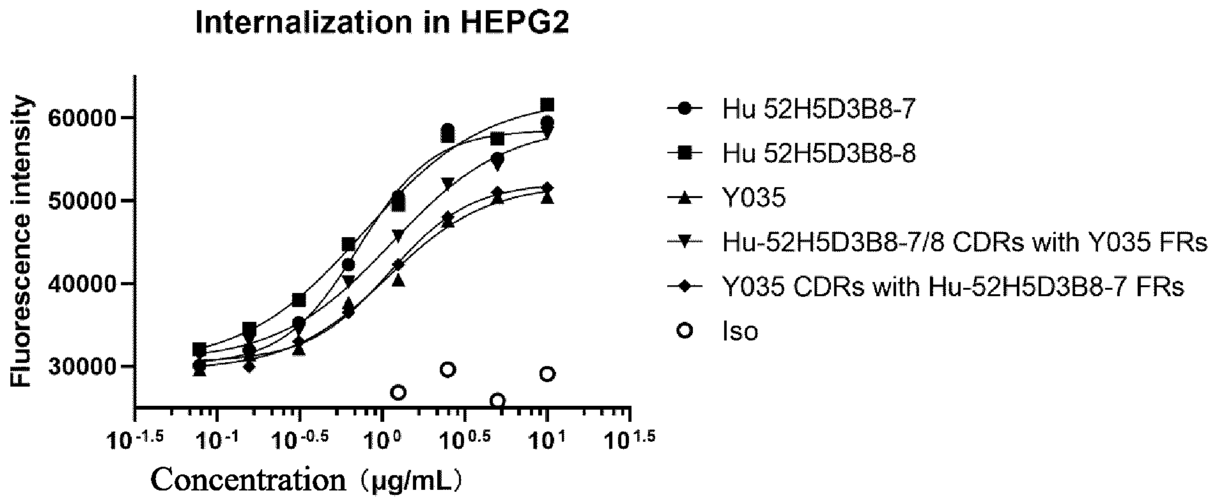
Internalization in HEPG2
Fluorescence intensity
60000
50000
40000
30000
$10^{-1.5}$ $10^{-1}$ $10^{-0.5}$ $10^{0}$ $10^{0.5}$ $10^{1}$ $10^{1.5}$
Concentration (μg/mL)
Hu 52H5D3B8-7
Hu 52H5D3B8-8
Y035
Hu-52H5D3B8-7/8 CDRs with Y035 FRs
Y035 CDRs with Hu-52H5D3B8-7 FRs
Iso

*FIG. 4*

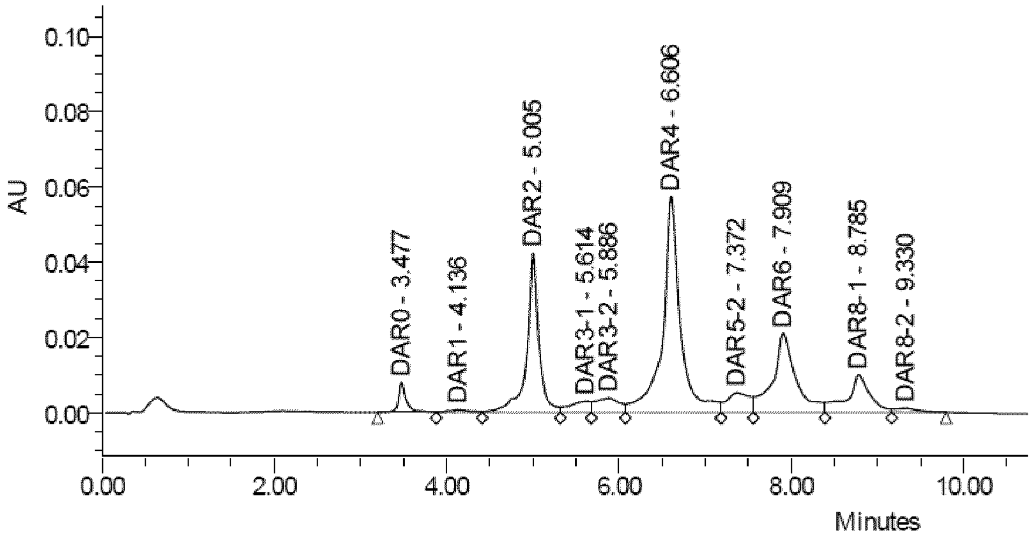
AU
0.10
0.08
0.06
0.04
0.02
0.00
DAR0 - 3.477
DAR1 - 4.136
DAR2 - 5.005
DAR3-1 - 5.614
DAR3-2 - 5.886
DAR4 - 6.606
DAR5-2 - 7.372
DAR6 - 7.909
DAR8-1 - 8.785
DAR8-2 - 9.330
0.00
2.00
4.00
6.00
8.00
10.00
Minutes

*FIG. 5*

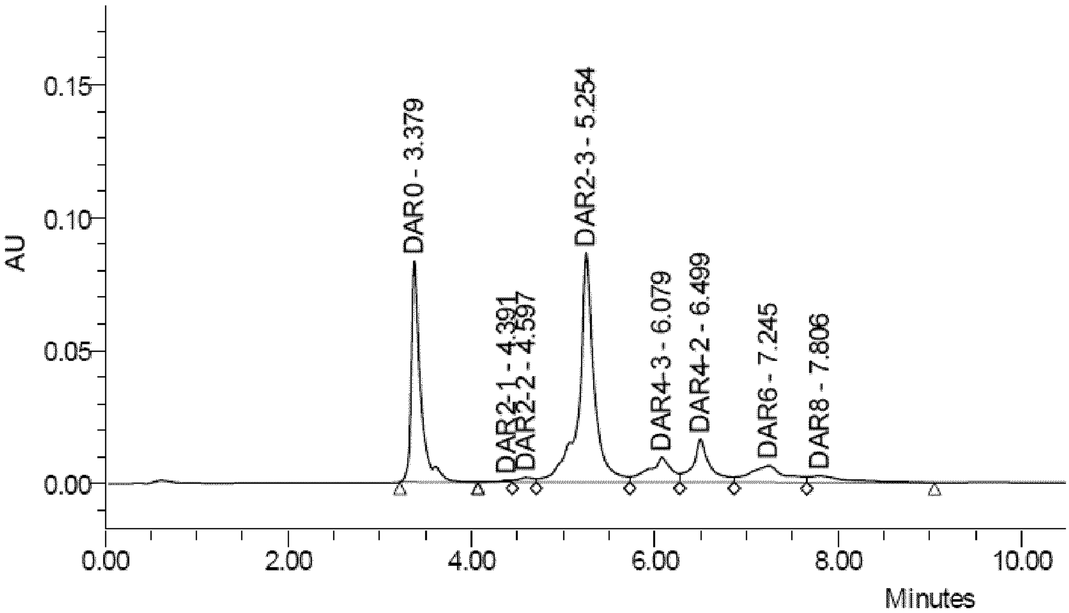
AU
0.15
0.10
0.05
0.00
DAR0 - 3.379
DAR2-1 - 4.391
DAR2-2 - 4.597
DAR2-3 - 5.254
DAR4-3 - 6.079
DAR4-2 - 6.499
DAR6 - 7.245
DAR8 - 7.806
0.00
2.00
4.00
6.00
8.00
10.00
Minutes

*FIG. 6*

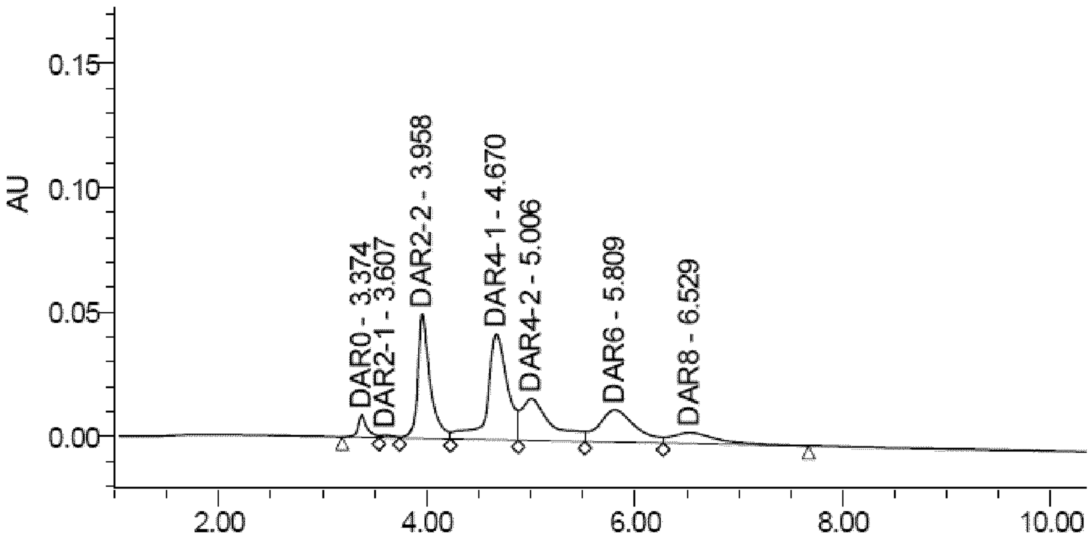
AU
0.15
0.10
0.05
0.00
DAR0 - 3.374
DAR2-1 - 3.607
DAR2-2 - 3.958
DAR4-1 - 4.670
DAR4-2 - 5.006
DAR6 - 5.809
DAR8 - 6.529
2.00
4.00
6.00
8.00
10.00

*FIG. 7*

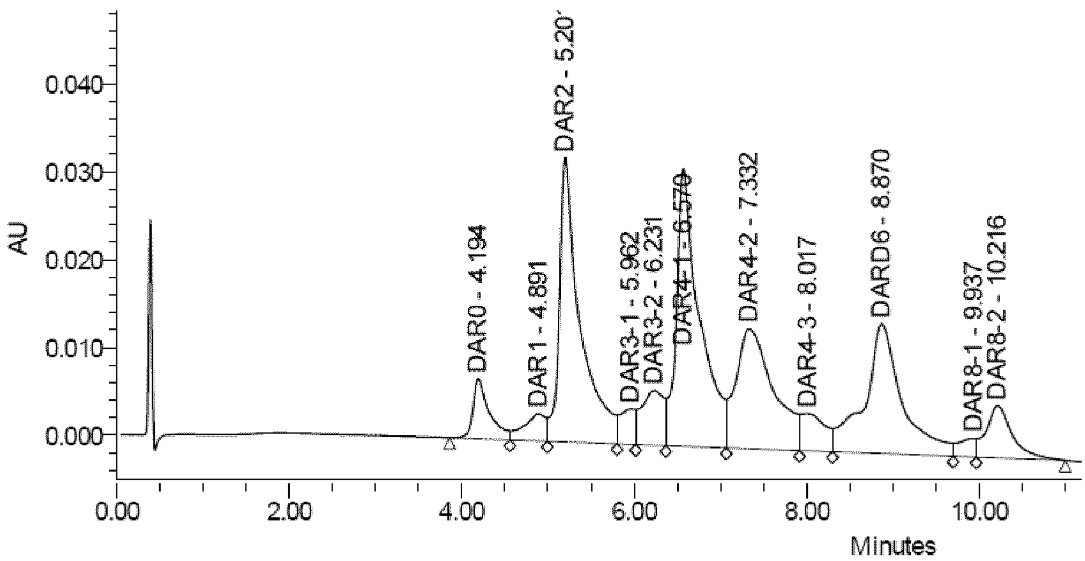
AU
Minutes
DAR0 - 4.194
DAR1 - 4.891
DAR2 - 5.20
DAR3-1 - 5.962
DAR3-2 - 6.231
DAR4-1 - 6.570
DAR4-2 - 7.332
DAR4-3 - 8.017
DARD6 - 8.870
DAR8-1 - 9.937
DAR8-2 - 10.216

*FIG. 8*

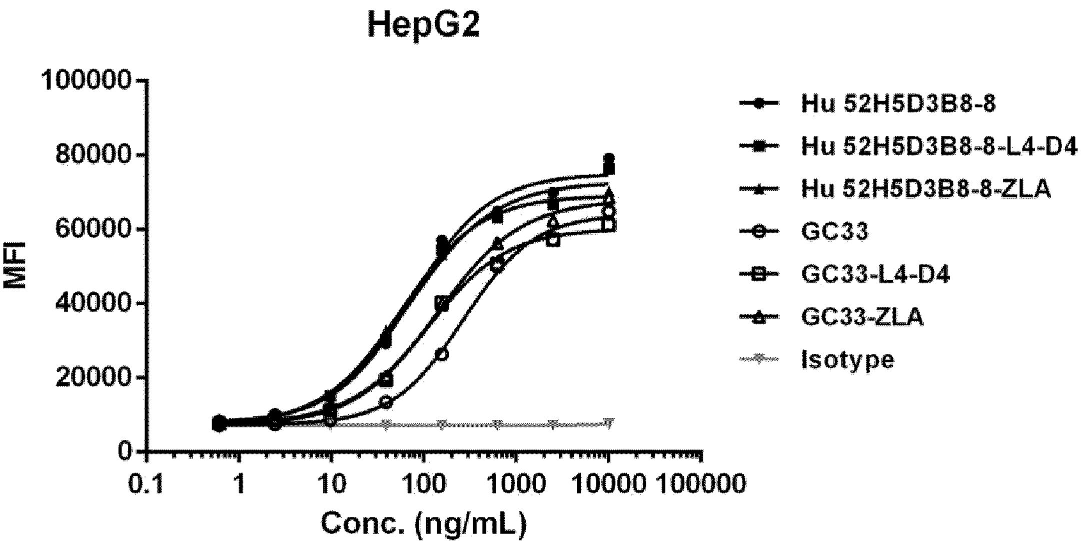
HepG2
MFI
Conc. (ng/mL)
Hu 52H5D3B8-8
Hu 52H5D3B8-8-L4-D4
Hu 52H5D3B8-8-ZLA
GC33
GC33-L4-D4
GC33-ZLA
Isotype

*FIG. 9*

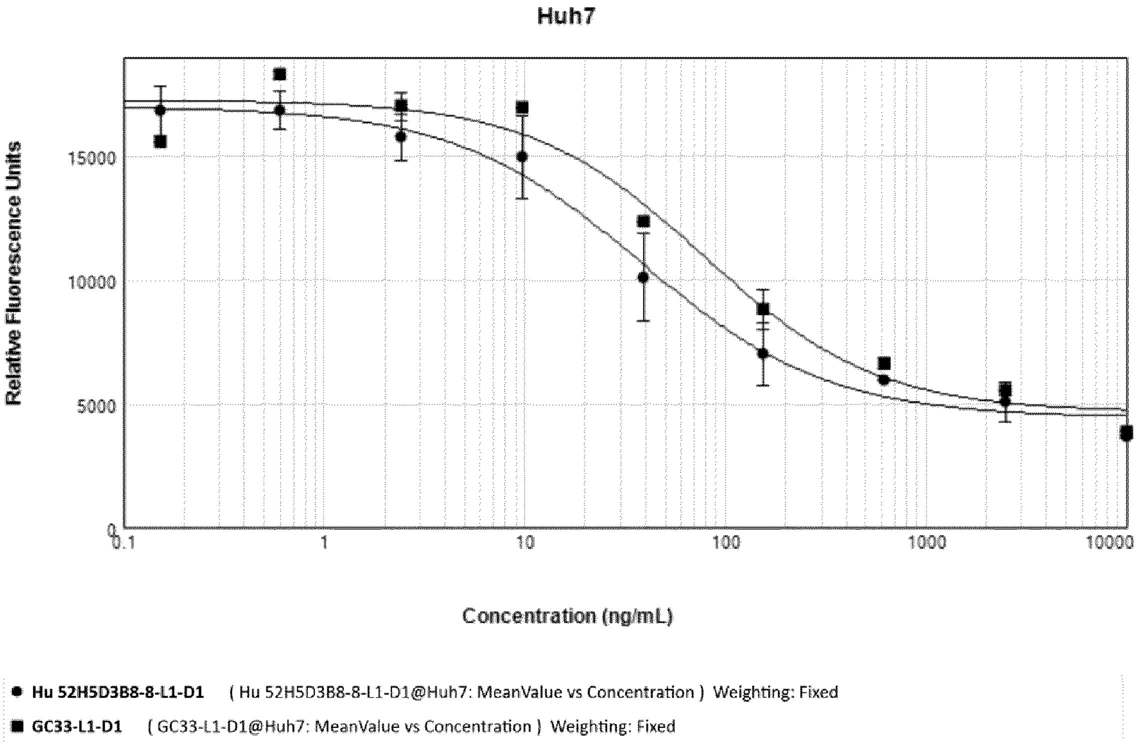
Huh7
Relative Fluorescence Units
15000
10000
5000
0
0.1
1
10
100
1000
10000
Concentration (ng/mL)
● Hu 52H5D3B8-8-L1-D1 ( Hu 52H5D3B8-8-L1-D1@Huh7: MeanValue vs Concentration ) Weighting: Fixed
■ GC33-L1-D1 ( GC33-L1-D1@Huh7: MeanValue vs Concentration ) Weighting: Fixed

*FIG. 10*

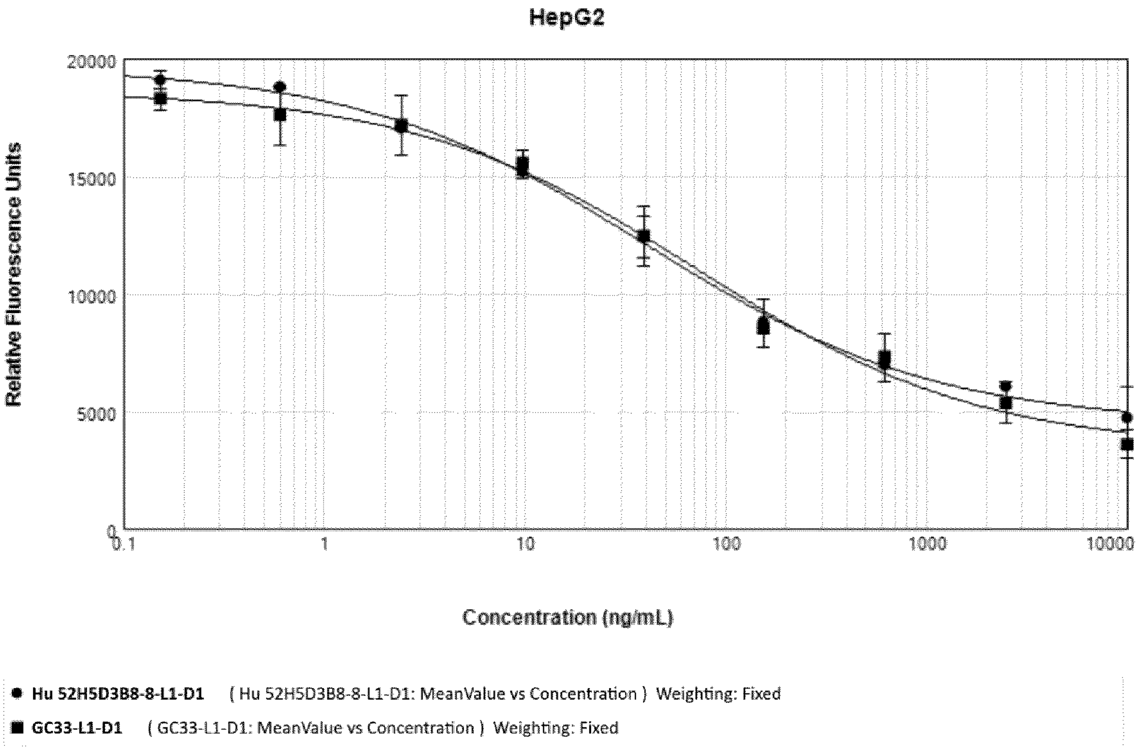
HepG2
Relative Fluorescence Units
20000
15000
10000
5000
0
0.1
1
10
100
1000
10000
Concentration (ng/mL)
● Hu 52H5D3B8-8-L1-D1 ( Hu 52H5D3B8-8-L1-D1: MeanValue vs Concentration ) Weighting: Fixed
■ GC33-L1-D1 ( GC33-L1-D1: MeanValue vs Concentration ) Weighting: Fixed

*FIG. 11*

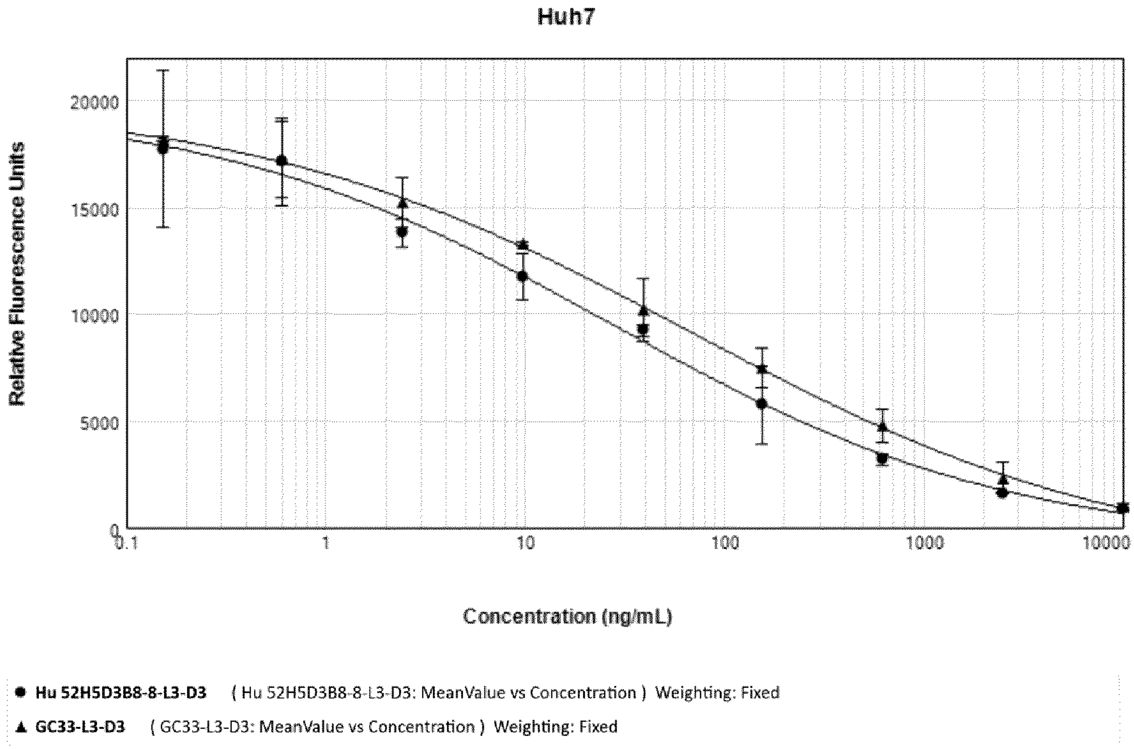
Huh7
Relative Fluorescence Units
20000
15000
10000
5000
0
0.1
1
10
100
1000
10000
Concentration (ng/mL)
Hu 52H5D3B8-8-L3-D3 ( Hu 52H5D3B8-8-L3-D3: MeanValue vs Concentration ) Weighting: Fixed
GC33-L3-D3 ( GC33-L3-D3: MeanValue vs Concentration ) Weighting: Fixed

*FIG. 12*

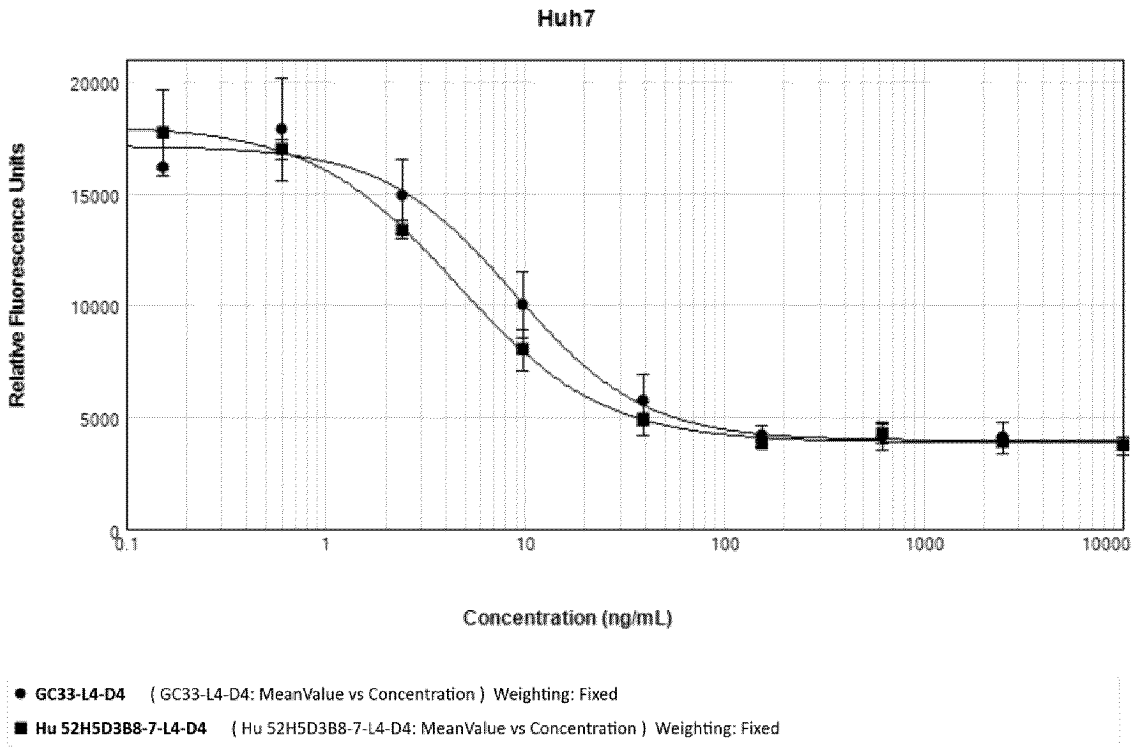
Huh7
Relative Fluorescence Units
20000
15000
10000
5000
0
0.1
1
10
100
1000
10000
Concentration (ng/mL)
GC33-L4-D4 ( GC33-L4-D4: MeanValue vs Concentration ) Weighting: Fixed
Hu 52H5D3B8-7-L4-D4 ( Hu 52H5D3B8-7-L4-D4: MeanValue vs Concentration ) Weighting: Fixed

*FIG. 13*

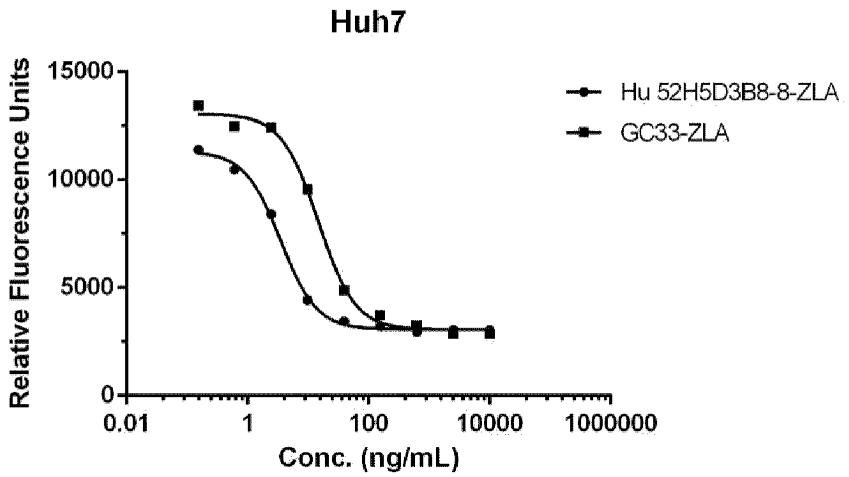
Huh7
Relative Fluorescence Units
15000
10000
5000
0
0.01
1
100
10000
1000000
Conc. (ng/mL)
Hu 52H5D3B8-8-ZLA
GC33-ZLA

*FIG. 14*

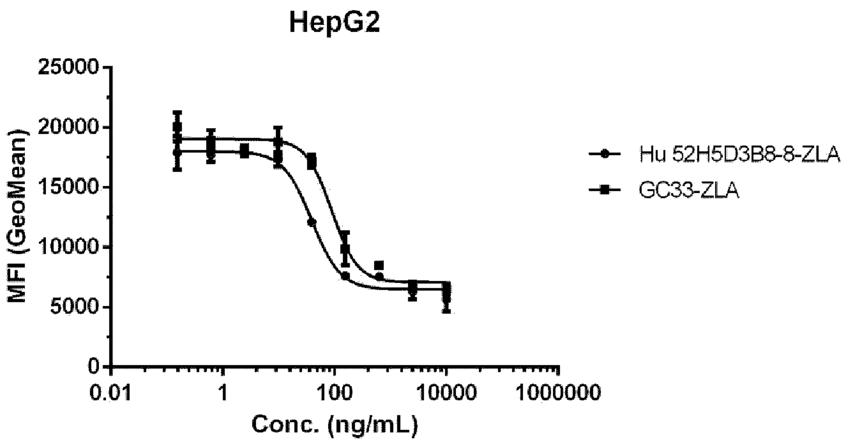
HepG2
MFI (GeoMean)
25000
20000
15000
10000
5000
0
0.01
1
100
10000
1000000
Conc. (ng/mL)
Hu 52H5D3B8-8-ZLA
GC33-ZLA

*FIG. 15*

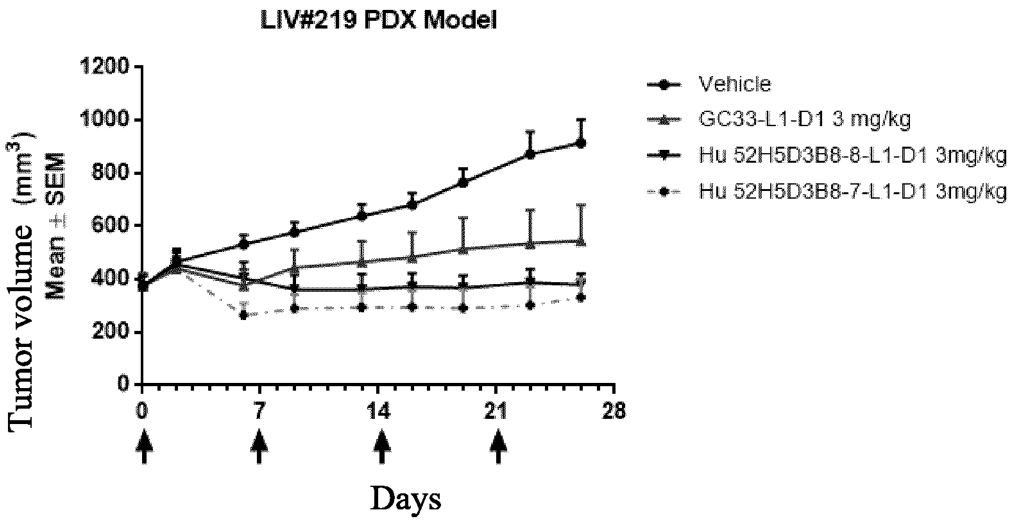
LIV#219 PDX Model
Tumor volume (mm3)
Mean ± SEM
1200
1000
800
600
400
200
0
0
7
14
21
28
Days
Vehicle
GC33-L1-D1 3 mg/kg
Hu 52H5D3B8-8-L1-D1 3mg/kg
Hu 52H5D3B8-7-L1-D1 3mg/kg

*FIG. 16*

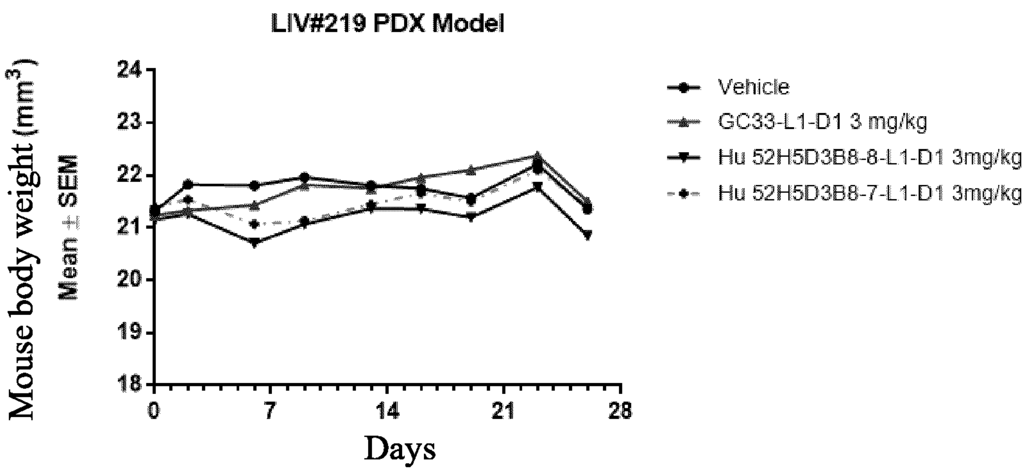
LIV#219 PDX Model
Mouse body weight (mm3)
Mean ± SEM
24
23
22
21
20
19
18
0
7
14
21
28
Days
Vehicle
GC33-L1-D1 3 mg/kg
Hu 52H5D3B8-8-L1-D1 3mg/kg
Hu 52H5D3B8-7-L1-D1 3mg/kg

*FIG. 17*

**INTERNATIONAL SEARCH REPORT**

International application No.
**PCT/CN2023/120286**

**A. CLASSIFICATION OF SUBJECT MATTER**

A61K47/68(2017.01)i; A61K45/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS; VEN; CNKI; NCBI; CNTXT; ENTXT; ISI web of science: 磷脂酰肌醇蛋白聚糖3, 抗体, 偶联, 药物, 毒素, MXR7, GTR2-2, DGSX, GPC3, Glypican-3, OCI-5, antibod+, drug, conjugat+; 中国专利生物序列检索系统, China Patent Biological Sequence Retrieval System; Genbank; EMBL, 检索的序列, retrieved sequence: SEQ ID NOs: 1-20等, SEQ ID NOs: 1-20 etc.

**C. DOCUMENTS CONSIDERED TO BE RELEVANT**

| Category* | Citation of document, with indication, where appropriate, of the relevant passages | Relevant to claim No. |
|---|---|---|
| A | CN 110577600 A (SHANGHAI INSTITUTE OF MATERIA MEDICA, CHINESE ACADEMY OF SCIENCES et al.) 17 December 2019 (2019-12-17)<br>see abstract, and claims 1-27 | 1-11 |
| A | CN 106414499 A (GENENTECH, INC.) 15 February 2017 (2017-02-15)<br>see abstract, and claims 1-73 | 1-11 |
| A | CN 111712520 A (SEATTLE GENETICS INC.) 25 September 2020 (2020-09-25)<br>see abstract, and claims 1-59 | 1-11 |
| A | CN 104520331 A (US DEPARTMENT OF HEALTH AND HUMAN SERVICES) 15 April 2015 (2015-04-15)<br>see abstract, and embodiments 1-5 | 1-11 |
| A | CN 114366818 A (SHANGHAI MIRACOGEN INC. et al.) 19 April 2022 (2022-04-19)<br>see abstract, and claims 1-15 | 1-11 |

☐ Further documents are listed in the continuation of Box C.    ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 December 2023** | **29 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/120286**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/120286**

| Patent document cited in search report | Publication date (day/month/year) | Patent family member(s) | Publication date (day/month/year) |
|---|---|---|---|
| CN 110577600 A | 17 December 2019 | None | |
| CN 106414499 A | 15 February 2017 | KR 20170003582 A | 09 January 2017 |
| | | RU 2016150370 A | 26 June 2018 |
| | | WO 2015179658 A2 | 26 November 2015 |
| | | WO 2015179658 A3 | 21 January 2016 |
| | | US 2018312602 A1 | 01 November 2018 |
| | | MX 2016015162 A | 03 March 2017 |
| | | EP 3145952 A2 | 29 March 2017 |
| | | CA 2946662 A1 | 26 November 2015 |
| | | BR 112016027222 A2 | 30 January 2018 |
| | | JP 2017522861 A | 17 August 2017 |
| CN 111712520 A | 25 September 2020 | AR 114112 A1 | 22 July 2020 |
| | | KR 20200121317 A | 23 October 2020 |
| | | US 2021361776 A1 | 25 November 2021 |
| | | US 11819553 B2 | 21 November 2023 |
| | | SG 11202007073 SA | 28 August 2020 |
| | | IL 276514 A | 30 September 2020 |
| | | JP 2023089195 A | 27 June 2023 |
| | | MX 2020008030 A | 10 September 2020 |
| | | CA 3090251 A1 | 22 August 2019 |
| | | EP 3752534 A1 | 23 December 2020 |
| | | EP 3752534 A4 | 01 December 2021 |
| | | WO 2019161174 A1 | 22 August 2019 |
| | | JP 2021513844 A | 03 June 2021 |
| | | TW 201938587 A | 01 October 2019 |
| | | AU 2019220700 A1 | 24 September 2020 |
| CN 104520331 A | 15 April 2015 | US 2015147330 A1 | 28 May 2015 |
| | | US 9409994 B2 | 09 August 2016 |
| | | WO 2013181543 A1 | 05 December 2013 |
| | | WO 2013181543 A8 | 31 December 2014 |
| | | SG 11201407972 RA | 29 January 2015 |
| | | KR 20150014521 A | 06 February 2015 |
| | | KR 102159773 B1 | 28 September 2020 |
| | | JP 2019062910 A | 25 April 2019 |
| | | JP 2015526387 A | 10 September 2015 |
| | | JP 6494507 B2 | 03 April 2019 |
| CN 114366818 A | 19 April 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7919086 B2
- CN 106397593 B
- CN 107001415 B


### Non-patent literature cited in the description


- Kabat Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- **ALTSCHUL et al.** *Nucl. Acid. Res.*, 1977, vol. 25, 3389-3402
- **ALTSCHUL et al.** *J. Mol. Biol.*, 1990, vol. 215, 403-410
- REMINGTON' S PHARMACEUTICAL SCIENCES. Mack Publishing Company, 1995
- Immunology-A Synthesis. Sinauer Associates, 1991